(19)

Europäisches
Patentamt

European
Patent Office

Office européen
des brevets

(11) **EP 2 166 358 A1**

(12) **EUROPEAN PATENT APPLICATION**

(43) Date of publication:
**24.03.2010 Bulletin 2010/12**

(51) Int Cl.:
*G01N 33/68* (2006.01)

(21) Application number: **08164495.7**

(22) Date of filing: **17.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Fundacio Institut de Recerca de l'Hospital**
**Universitari Vall d'Hebron**
**08035 Barcelona (ES)**

(72) Inventor: **Montaner Vilallonga, Joan**
**08037 Barcelona (ES)**

(74) Representative: **ABG Patentes, S.L.**
**Avenida de Burgos 16D**
**Edificio Euromor**
**28036 Madrid (ES)**

(54) **Differential diagnostic biomarkers of stroke mimicking conditions and methods of use thereof**

(57)   The present invention relates to the identification and use of diagnostic markers related with brain damage, particularly, biomarkers independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, that are associated with the diagnosis, prognosis, or differentiation of stroke mimicking conditions and stroke in a subject.

EP 2 166 358 A1

Printed by Jouve, 75001 PARIS (FR)

**Description**

**FIELD OF THE INVENTION**

The present invention relates to the identification and use of diagnostic markers related with brain damage, particularly, biomarkers that are associated with the diagnosis, prognosis, or differentiation of stroke mimicking conditions and stroke in a subj ect.

**BACKGROUND OF THE INVENTION**

Stroke is a manifestation of vascular injury to the brain which is commonly secondary to atherosclerosis or hypertension, is the third leading cause of death (and the second most common cause of neurologic disability) and the most common cause of permanent disability in adults worldwide. It is a significant socioeconomic burden for all countries and responsible for up to 4% of the total health care costs.

Stroke can be categorized into two broad types, "ischemic stroke" and "hemorrhagic stroke". Additionally, a patient may experience transient ischemic attacks, which are in turn a high risk factor for the future development of a more severe episode.

There are also conditions which mimic stroke ("stroke mimicking conditions"), which are not true stroke, which must be properly diagnosed in order to administer a therapy compatible with the diagnosis to the patient. The most frequent causes of stroke mimicking conditions have been established by Nor et al. and by Hand et al. and are described in those generically known as the "ROSIER study" [Nor AM et al. The Recognition of Stroke in the Emergency Room (ROSIER) scale: development and validation of a stroke recognition instrument. Lancet Neurol. 2005 Nov; 4(11):727-34)] and the "Brain Attack Study" [Hand PJ et al. Distinguishing between stroke and mimic at the bedside: the brain attack study. Stroke. 2006 Mar; 37(3):769-75], respectively. In general, the majority of stoke mimicking conditions use to be postictal states (seizures), tumors, migraine, systemic infections and toxic-metabolic disturbances.

Usual diagnostic methods for stroke includes procedures such as non-contrast computed tomography (CT) scan, electrocardiogram, magnetic resonance imaging (MRI), and angiography, some of them being costly and time-consuming. However, identifying if a patient suffers from a real stroke as well as determining the immediate cause of stroke in order to administer the suitable therapy is oftenly difficult by these means. CT scan often cannot detect ischemic strokes until 6 hours from onset, depending on the infarct size. MRI may be more effective than CT scan in early detection of ischemic stroke, but it is less accurate at differentiating ischemic from hemorrhagic stroke, it does not reach 100% specificity and is not widely available. An electrocardiogram (ECG) can be used in certain circumstances to identify a cardiac cause of stroke. Angiography is a definitive test to identify stenosis or occlusion of large and small cranial blood vessels, and can locate the cause of subarachnoid hemorrhages, define aneurysms, and detect cerebral vasospasm; however, it is an invasive procedure that is also limited by cost and availability. Coagulation studies can also be used to rule out a coagulation disorder (coagulopathy) as a cause of hemorrhagic stroke.

Since immediate diagnosis and care of a patient experiencing stroke is critical, it is very important to differentiate true stroke from stroke mimicking conditions. For example, tissue plasminogen activator (tPA) given within three hours of symptom onset in ischemic stroke is beneficial for selected acute stroke patients. Alternatively, patients may benefit from anticoagulants (e.g., heparin) or antiplatelet agents if they are not candidates for TPA therapy. Thus, early differentiation of true stroke and stroke mimicking conditions is imperative. In fact, delays in the confirmation of true stroke diagnosis and the identification of stroke type limit the number of patients that may benefit from early intervention therapy.

Consequently, since admission to a stroke unit and thrombolysis within 3 hours, or even within 6 hours when MRI is available, offer a great benefit to stroke patients, most patients with sudden neurological deficit are treated as medical emergencies. However, it is well known that non-cerebrovascular conditions can present with a clinical picture mimicking stroke, so, early accurate differentiation of such stroke mimicking conditions from true stroke is essential.

Although to send all this stroke suspicions to a referral center might be a possibility in order to offer all patients the opportunity of getting benefit of those stroke units and suitable treatment, it seems more reasonable to rule-out stroke-mimicking conditions from acute ischemic stroke quickly and accurately in order to avoid unnecessary urgent transfers, specialist evaluation and extra-testing.

At present, the absence of a widely available diagnostic test for acute cerebral ischemia remains a limitation in the diagnosis and management of stroke. In spite of great improvements in the field, mainly the new treatments with TPA, still very few patients benefit from those treatments, and many of them will never arrive on time to get those drugs in hospitals able to give those stroke treatments. Therefore, a sensitive and specific diagnostic assay for stroke and stroke mimicking conditions which could also differentiate between stroke and stroke mimicking conditions might help in differentiating true stroke from stroke mimicking conditions and to aid in the management of said conditions.

The incidence of these mimics is reported to vary from 1.2% to 50% and depends on when and by whom the patient is investigated. The triaging stage is likely to have the highest rate of stroke mimicking conditions. Disorders considered by ambulance personnel to mimic stroke at the triaging stage may be considered no stroke at all by an experienced neurologist who also has laboratory testing and CT scan of the brain at his disposal.

Therefore that screening stage seems the best scenario for a quick, sensitive and specific diagnostic assay for stroke and stroke mimicking conditions which could also differentiate between stroke and stroke mimicking conditions that might be measured quickly offering a very high sensitivity to send to reference hospitals all true strokes that might get therapeutically benefit and keeping at those centers many of the stroke mimicking conditions.

For neurologists to evaluate the incidence of stroke mimicking conditions at the stage of deciding about stroke unit admission and thrombolytic therapy, might be also important although a very specific test seems more promising in that scenario as compared with a very sensitive test in the screening scenarios of ambulances, small hospitals or emergency departments. In fact, at a stage in which thrombolysis was already given also several patients were stroke mimicking conditions and therefore treatment should had never been given since it is a potential harmful drug used in wrong indications.

On the other side, the use of plasma biomarkers is getting increasingly popular in several fields of medicine. In fact, decision making processes using biomarkers is widely accepted in medical situations such as initiating lipid lowering therapies (LDL), diagnosing acute myocardial infarction (troponins), ruling-out pulmonary embolism suspicions (D-dimer), etc. Interest is also coming from cerebrovascular diseases, since biomarkers might help physicians in several steps of stroke evaluation. At first, biomarkers were mainly used to determine the risk of stroke appearance. In fact, inflammatory markers, such as high sensitivity C-reactive protein (hs-CRP), have been associated with recurrence of vascular events following stroke. That is also the case of the recently FDA-approved lipoprotein-associated phospholipase A2, PLAC test, that predicts the risk of stroke appearance among healthy individuals. This kind of approaches permits to use more aggressive secondary prevention treatments.

In recent years, new utilities have been identified for plasma biomarkers, mainly in the acute stroke field and that is the idea of getting a biochemical stroke diagnosis. A biomarker-based exclusion strategy will result in health economic advantages in terms of cost savings on imaging and reduced length of stay.

Decision making in patients presenting with symptoms suspicious for stroke is challenging, particularly in the first hours after symptom onset, where brain imaging is most often not conclusive and time windows very short to make important therapeutically decisions mainly related to the fact that ischemic stroke patients evaluated within 3 hours of stroke onset might be treated with thrombolytic drugs.

Although stroke biomarkers with a diagnosis application will not probably be used as a rule-in test, since confirmation by imaging may be desired, biomarkers will be more cost-effective as rule-out test, to exclude patients attending the emergency room with signs and symptoms suggestive of stroke. Thus, the test should be optimized for sensitivity to guarantee a high negative predictive value.

Several authors are trying to evaluate the value of diagnostic panels of blood-borne biochemical markers of cerebral ischemia. Although the ideal scenario for specific biomarkers is to differentiate "real strokes" from "other causes mimicking stroke (mimics)", until now research work has mainly focused on biomarkers that distinguish stroke patients from healthy controls, in that kind of studies, tested biomarkers have been for example: S-100B (a marker of astrocytic activation), B-type neurotrophic growth factor, von Willebrand factor, matrix metalloproteinase-9, and monocyte chemotactic protein-1. In a panel algorithm in which three or more marker values above their respective cut-offs were scored as positive, these five markers provided a sensitivity of 92% at 93% specificity for ischemic stroke samples taken within 6 h from symptom onset. Another study from the same group (Biosite and collaborators), analyzed 26 blood-borne markers, and revealed that 4 were highly correlated with stroke (P<0.001): a marker of glial activation (S100B), 2 markers of inflammation (matrix metalloproteinase-9 and vascular cell adhesion molecule), and 1 marker of thrombosis (von Willebrand factor). The logistic model provided a sensitivity and specificity of 90% for predicting stroke.

These data suggest that to use a combination of biomarkers seems promising to make an urgent biochemical diagnosis of stroke. If true, this approach would permit rapid referral of stroke patients to hospitals with acute treatments available, as commented in the previous sections.

Moreover, biomarkers might go further in the aim of differentiating stroke subtypes. Although brain CT is performed as part of the initial evaluation of a patient with suspected stroke in most hospitals, because of its sensitivity to differentiate ischemic from hemorrhagic stroke, the availability of a rapid biochemical diagnostic test would add clear advantages in the management of stroke patients. Some authors have already described biomarkers aiding in this direction such as glial fibrillary acidic protein (GFAP). Serum GFAP was substantially raised in patients with ICH; in fact authors identified that GFAP was detectable in the serum of 81% of patients with ICH, and only in 5% patients with ischemic stroke. A cut off point of 2.9 ng/1 provided a sensitivity of 0.79 and a specificity of 0.98 for the identification of ICH, making these kinds of tests very attractive for pre-hospital assessment.

Proteomics seems a promising tool for massive biomarkers identification in the stroke field, mainly directed to diagnosis and treatment response. In fact, several biomarkers have been identified from 2-dimensional electrophoresis gels of postmortem CSF, which were compared with gels from ante mortem CSF by the Geneva group. In that study, the identification of PARK7 and NDKA was based on their increased concentrations in postmortem CSF, presumably as a result of global brain ischemia and necrosis after death.

US2004/0219509 discloses a method of diagnosing stroke in a subject, which comprises determining the presence or amount of a plurality of subject-derived markers in a sample obtained from said subject, wherein said plurality of markers are independently selected from two or more members of the group consisting of specific markers of neural tissue injury, markers related to blood pressure regulation, markers related to coagulation and hemostasis, and markers related to inflammation, and markers related to apoptosis; and correlating the presence or amount of said plurality of markers to the occurrence of a stroke in said subject.

However, no biomarker has shown to accurately differentiate strokes from stroke mimicking conditions. In fact, there are no publications using this approach and most of the "stroke biomarkers" publications compare those markers with healthy controls.

Accordingly, there is a present need in the art for a rapid, sensitive and specific diagnostic assay for stroke and stroke mimicking conditions that can also differentiate between stroke and stroke mimicking conditions. Such a diagnostic assay would reduce the costs associated with incorrect stroke diagnosis and greatly increase the number of patients that can receive beneficial stroke therapies.

## SUMMARY OF THE INVENTION

The present invention relates to the identification and use of diagnostic biomarkers related with brain damage, particularly, biomarkers capable to differentiate stroke mimicking conditions from stroke. The methods described herein can meet the need in the art for rapid, sensitive and specific diagnostic assay to be used in the diagnosis and differentiation of stroke mimicking conditions from stroke. Moreover, the methods of the present invention can also be used to facilitate the treatment of subjects suffering from stroke mimicking conditions or the treatment of stroke patients, as well as the development of additional diagnostic and/or prognostic indicators.

Therefore, in general, the invention relates to methods for identifying markers that are associated with the diagnosis, prognosis, or differentiation of stroke mimicking conditions and stroke in a subject; to using such markers in diagnosing and treating a subject and/or to monitor the course of a treatment regimen; to using such markers to identify subjects at risk for one or more adverse outcomes related to stroke mimicking conditions and/or stroke; and for screening compounds and pharmaceutical compositions that might provide a benefit in treating or preventing such conditions.

Thus, in an aspect, the invention discloses a method for diagnosing a stroke mimicking condition in a subject by determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, and comparing said levels with the levels of the same biomarkers in a reference sample, wherein said reference sample is a stroke-positive sample.

In another aspect, the invention relates to a method for diagnosing stroke in a subject by determining the level of a subject-derived biomarker in a sample obtained from said subject, wherein said biomarker is independently selected from the group consisting of IL-17, FasL, bNGF, IGFBP3, TNFR-1, GroA and combinations thereof, and comparing said levels with the levels of the same biomarkers in a reference sample, wherein said reference sample is a stroke mimicking condition-positive sample.

In another aspect, the invention relates to a method for the determination of the efficacy of a therapy for a stroke mimicking condition in a subject.

In another aspect, the invention relates to a method for the determination of the efficacy of a therapy for stroke in a subject.

In another aspect, the invention relates to a method for the identification of compounds suitable for the treatment of stroke.

In another aspect, the invention relates to a method for the identification of compounds suitable for the treatment of a stroke mimicking condition.

In another aspect, the invention relates to a method for the differential diagnosis of stroke or stroke mimicking conditions in a subject.

In another aspect, the invention relates to a kit comprising a reagent or a set of reagents for the analysis of a biomarker selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof The use of said kit in the diagnosis and/or prognosis of stroke or a stroke mimicking condition constitutes a further aspect of the present invention.

## BRIEF DESCRIPTION OF THE DRAWINGS

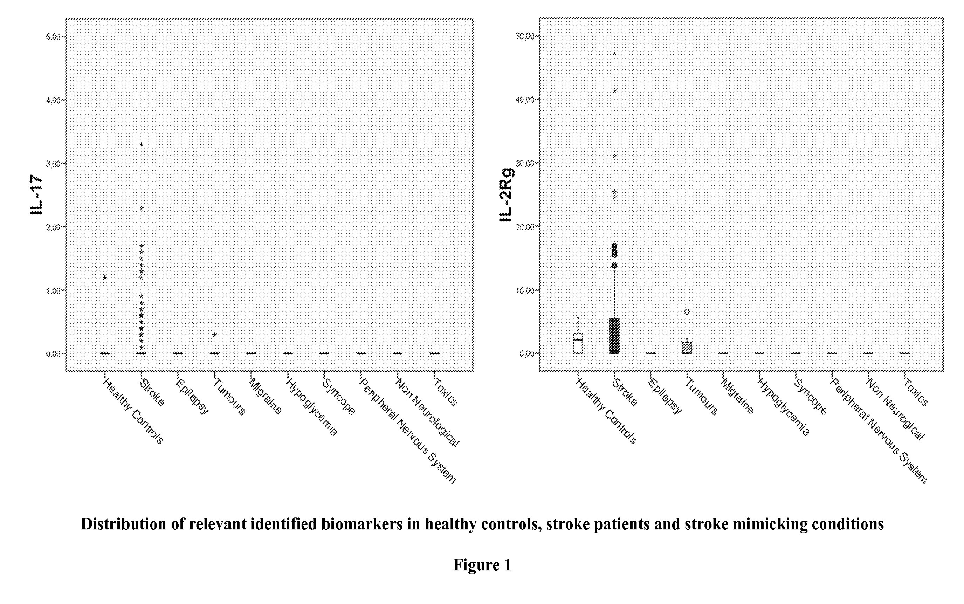

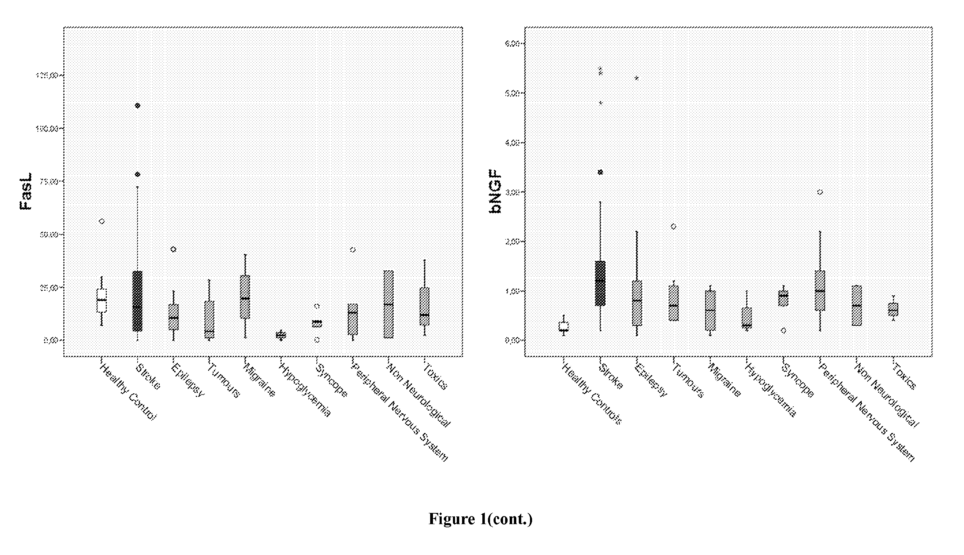

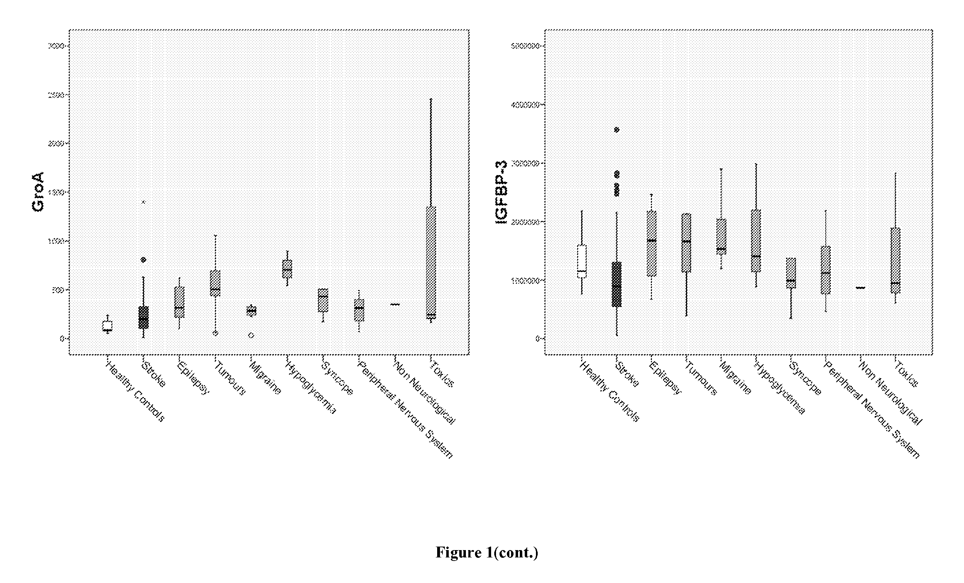

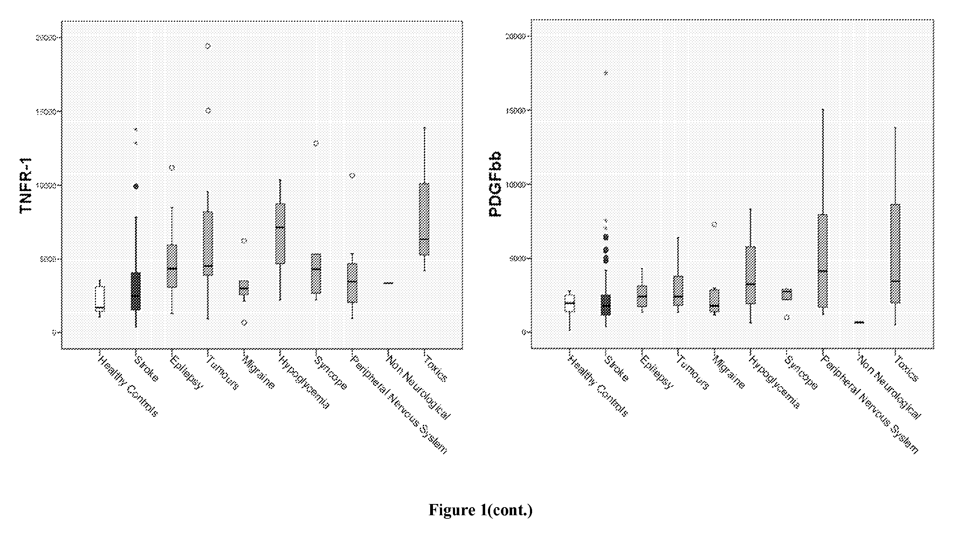

Figure 1 is a set of graphics showing the distribution of relevant biomarkers (IL-17, IL2RG, FasL, bNGF, GroA, IGFBP3, TNFR1, and PDGFBB) among different studied groups of patients (healthy controls, stroke patients and stroke mimics conditions).

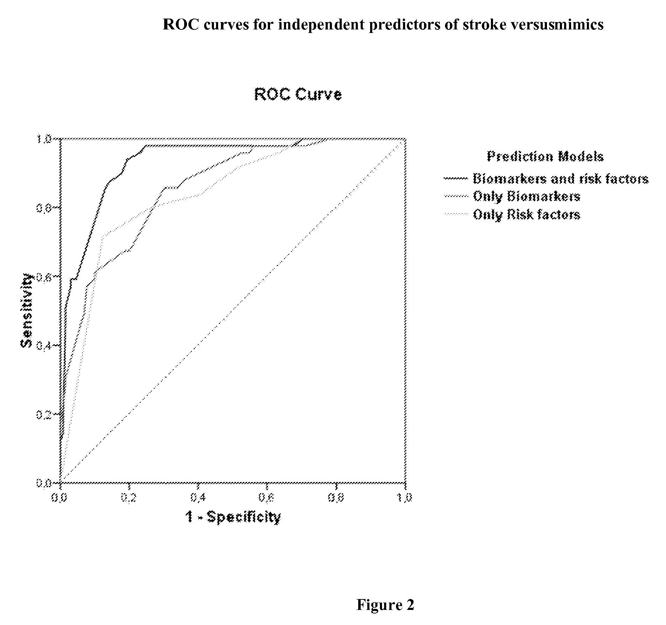

Figure 2 is a graphic showing ROC curves for independent predictors of stroke patients versus stroke mimic subjects. Three ROC curves were constructed comparing diagnostic accuracy of selected biomarkers (IL-17, FasL, bNGF, GroA, IGFBP3 and TNFR1), selected clinical variables (Hypertension, Atrial Fibrillation, Dyslipidemia or Smoking Tobacco) or the combination of both biomarkers and clinical variables (IL-17, bNGF, IGFBP3, TNF1, Hypertension, Atrial Fibrillation and Dyslipidemia).

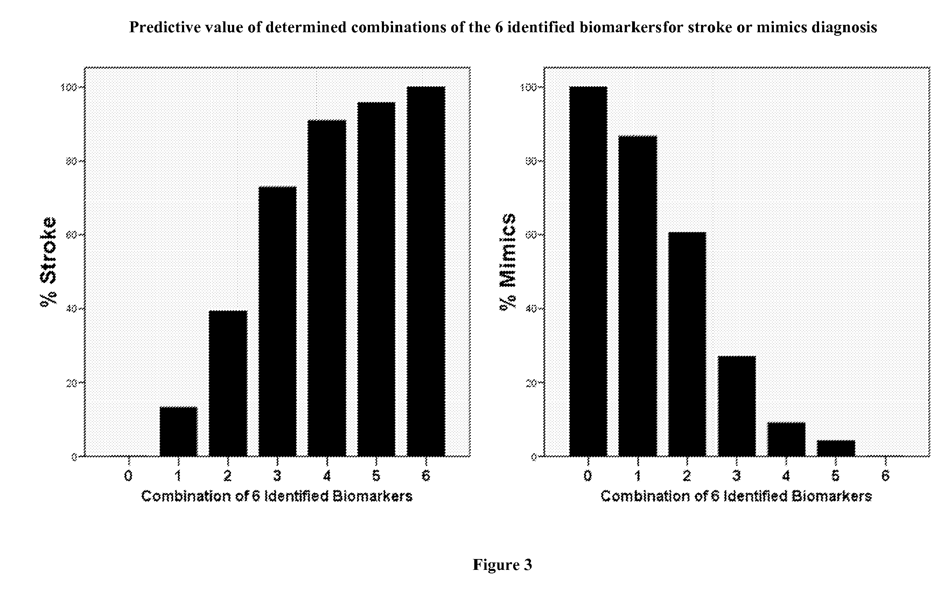

Figure 3 is a set of two graphics, one showing the results of a model with 6 markers (3 high and 3 low markers for stroke) provided by the instant invention that are independent biomarkers of stroke versus stroke mimicking conditions; some combinations achieving sensitivities of 100% and specificities of 100%, and the other one showing the results of a model with 6 markers (3 high and 3 low markers for mimics) that are independent biomarkers of mimics versus stroke; some combinations achieving sensitivities of 100% and specificities of 100%.

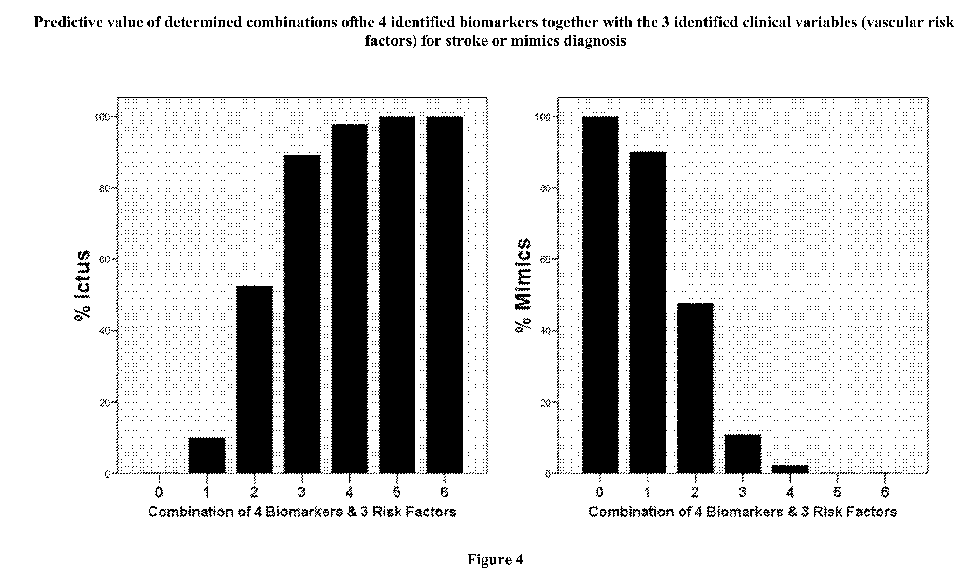

Figure 4 is a set of two graphics, one showing the results of a model with 4 biomarkers (2 high and 2 low markers for stroke, that are IL-17, bNGF, IGFBP3, and TNFR1 respectively) and 3 clinical variables (present among stroke patients, that are Hypertension, Atrial Fibrillation and Dyslipidemia) that are independent predictors of stroke versus stroke mimicking conditions; some combinations achieving sensitivities of 100% and specificities of 100% and one showing the results of a model with 4 biomarkers (2 high and 2 low markers for mimics that are IGFBP3, TNF1, IL-17 and bNGF, respectively) and three clinical variables (absent among stroke mimic subjects (mimic patients) that are Hypertension, Atrial Fibrillation and Dyslipidemia) that are independent predictors of stroke mimicking conditions versus stroke; some combinations achieving sensitivities of 100% and specificities of 100%.

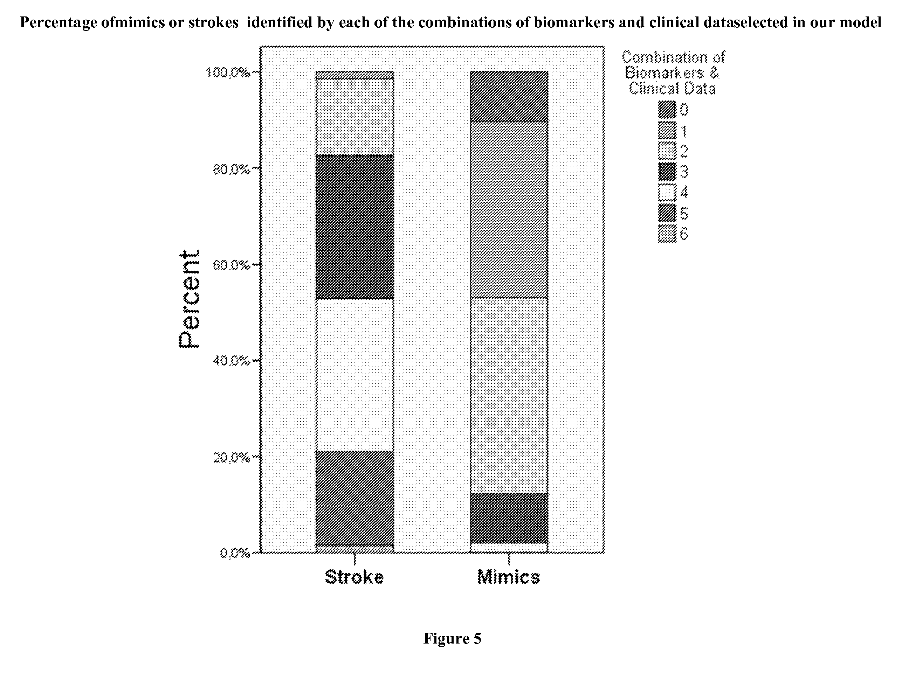

Figure 5 is a graphic showing that combining information coming from some clinical variables and biomarkers (IL-17, bNGF, IGFBP3, TNFR1, Hypertension, Atrial Fibrillation and Dyslipidemia) selected in a multivariate analysis allowed building up a model with a great predictive value to screen stroke patients.

## DETAILED DESCRIPTION OF THE INVENTION

In accordance with the present invention, there are provided methods and compositions for the identification and use of biomarkers related with brain damage, particularly, biomarkers that are associated with the diagnosis, prognosis, or differentiation of stroke and stroke mimicking conditions in a subject. Such biomarkers can be used in diagnosing and treating a subject and/or to monitor the course of a treatment regimen; for screening subjects for the occurrence of a particular disease; and for screening compounds and pharmaceutical compositions that might provide a benefit in treating or preventing such conditions. Further, the predictive value for stroke mimicking conditions and for real strokes using these biomarkers based model is very convenient for being used to refer patients to stroke hospitals as a screening tool.

## I. Diagnosis of stroke mimicking conditions

As it has been previously mentioned, the inventors have identified some biomarkers present in the serum or plasma of subjects who suffer from one or more stroke-associated symptoms but that have not suffered from a stroke (i.e., stroke mimic subjects suffering from a stroke mimicking condition) which are present at different levels with respect to the serum or plasma of subjects who suffer or have suffered from a stroke; consequently, said biomarkers can then be used, for example, in a rapid, sensitive and specific diagnostic assay to be used in the diagnosis and differentiation of stroke mimicking conditions from stroke.

Thus, in an aspect, the invention relates to a method (hereinafter "the first method of the invention") for diagnosing a stroke mimicking condition in a subject, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the subject is diagnosed as having a stroke mimicking condition when the levels of one or more of biomarkers selected from IL-17, FasL, bNGF and combinations thereof, is/are decreased with respect to the level of the same biomarker in a reference sample and/or the levels of one or more biomarkers selected from IGFBP3, TNFR1, GroA and combinations thereof is/are increased with respect to the level of the same biomarker in a reference sample, wherein said reference sample is a stroke-positive sample.

The expression "method for diagnosing" as referred to in accordance with the present invention means that the method may essentially consist of the aforementioned steps or may include further steps. However, it is to be understood that the method, in a preferred embodiment, is a method carried out *in vitro,* i.e. not practiced on the human or animal body. "Diagnosing", as it is used herein, refers to assessing the probability according to which a subject is suffering from a disease. As it will be understood by those skilled in the art, such an assessment, although preferred to be, may usually not be correct for 100% of the subjects to be diagnosed. The term, however, requires that a statistically significant portion of subjects can be identified as suffering from the disease. Whether a portion is statistically significant can be determined easily by the person skilled in the art by using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or even at least 95%. The p-values are, preferably, 0.2, 0.1 or 0.05.

The term "stroke", as used herein, relates to a pathological condition with acute onset that is caused by the occlusion or rupture of a vessel supplying blood, and thus oxygen and nutrients, to the brain. The immediate area of injury is referred to as the "core", which contains brain cells that have died as a result of ischemia or physical damage. The "penumbra" is composed of brain cells that are neurologically or chemically connected to cells in the core. Cells within the penumbra are injured, but still have the ability to completely recover following removal of the insult caused during stroke. However, as ischemia or bleeding from hemorrhage continues, the core of dead cells can expand from the site of insult, resulting in a concurrent expansion of cells in the penumbra. The initial volume and rate of core expansion is related to the severity of the stroke and, in most cases, neurological outcome. The term "stroke" includes any specific type of stroke (e.g., thrombotic, embolic, lacunar, hypoperfusion, intracerebral hemorrhage, and subarachnoid hemorrhage types of strokes). The confirmation of this condition state may be made through any suitable assay such as those mentioned in the "Background of the Invention", e.g., CT or MRI.

Specific neurologic dysfunctions or "stroke-associated symptoms" may include, but are not limited to, pain, headache, aphasia, apraxia, agnosia, amnesia, stupor, confusion, vertigo, coma, delirium, dementia, seizure, migraine insomnia, hypersomnia, sleep apnea, tremor, dyskinesia, paralysis, visual disturbances, diplopia, paresthesias, dysarthria, hemiplegia, hemianesthesia, hemianopia, etc. Subjects exhibiting one or more of these symptoms but that have not suffered from a stroke are referred to herein as "stroke mimic subjects" and the conditions they suffer are referred to herein as "stroke mimicking conditions". Illustrative, non limitative examples of stroke mimicking conditions include brain tumors (including primary and metastatic disease or other space occupying lesion), hypoglycemia, infections (e.g., meningitis), metabolic disorders, migraine, acute confusional state, multiple sclerosis, nerve palsy (cranial or peripheral), peripheral vascular disease, peripheral neuropathy, radiculopathy, seizure (including grand mal seizure), post-seizure paralysis, sepsis, stress, subdural hematoma, syncope/presyncope, toxic-metabolic disturbances, dehydration, encephalopathy, somatisation, labyrinthitis, vestibular dysfunction, electrocution, head injury (including concussion), dementia, orthostatic hypotension, arthropathy/arthritis, social breakdown, cervical myelopathy, medication side-effect, parkinsonism, vasculitis, aneurysm, bums, transient global amnesia and transient unilateral weakness. In a particular embodiment, said stroke mimicking conditions are selected from the group consisting of brain tumors, encephalopathy, labyrinthitis, migraine, neuropathy, radiculopathy, seizure (e.g., epileptic seizure), sepsis, somathisation, syncope, systemic infections, toxic-metabolic disturbances, dementia and transient global amnesia. Biomarkers and biomarker panels provided by this invention can distinguish these stroke mimicking conditions (stroke mimics) from true stroke.

The term "biomarker" as used herein refers to proteins or polypeptides to be used as targets for screening test samples obtained from subjects. "Proteins or polypeptides" used as biomarkers in the present invention are contemplated to include any fragments thereof, in particular, immunologically detectable fragments. The skilled person in the art will recognize that proteins which are released by cells of the central nervous system (CNS) or other parts of the body which become damaged during a cerebral attack could become degraded or cleaved into such fragments. Additionally, certain markers are synthesized in an inactive form, which may be subsequently activated, e.g., by proteolysis. The term "biomarker" also includes "related biomarker"; said term "related biomarker" as used herein refers to one or more fragments of a particular biomarker that may be detected as a surrogate for the biomarker itself. These related biomarkers may be, for example, "pre", "pro" or "prepro" forms of biomarkers, or the "pre", "pro" or "prepro" fragment removed to form the mature biomarker. In preferred embodiments, these "pre", "pro" or "prepro" forms or the removed "pre", "pro" or "prepro" fragments are used in an equivalent fashion to the mature biomarkers in the methods described herein.

According to the present invention, suitable biomarkers for the diagnosis of a stroke mimicking condition include IL-17, FasL, bNGF, IGFBP3, TNFR-1 and GroA. Each of these terms is defined hereinafter.

Interleukin-17 (IL-17) is a newly identified T-cell-specific cytokine. In the field of brain ischemia, IL-17 mRNA level was shown to be elevated in the ischemic hemispheres of pMCAO-operated rats compared with corresponding hemispheres of sham-operated rats. Levels were slightly elevated at 1 h, and peaked at 6 days after pMCAO. Systemically, the numbers of IL-17 mRNA expressing blood mononuclear cells were elevated already at 1 h after pMCAO, preceding the changes in the ischemic hemispheres [Li HL et al. IL-17 and IFN-gamma mRNA expression is increased in the brain and systemically after permanent middle cerebral artery occlusion in the rat. J Neuroimmunol. 2001; 116(1):5-14]. More recently, said research group evaluated the source and the action of IL-17 over the course of cerebral ischemia in rats and humans. The levels of IL-17 in the ischemic hemisphere of the human brain, which was removed at necropsy, were assayed immunohistochemically. Levels of IL-17 were elevated in the ischemic hemispheres of human brain compared with the opposite normal hemispheres and peaked at days 3-5 after brain ischemia. IL-17 mRNA was also elevated in ischemic hemispheres of pMCAO-operated rats, which were slightly elevated after 1 h and peaked at 6 days. Authors suggested that in additional to T cells the neuroglial cell may be another cellular origin of IL-17 in later progression of brain ischemia [Li GZ et al. Expression of interleukin-17 in ischemic brain tissue. Scand J Immunol. 2005 Nov; 62(5): 481-6]. In fact, it was known that patients with ischemic stroke had elevated numbers of IL-1b, IL-8, and IL-17 mRNA expressing PBMC 1 to 3 days after onset of symptoms compared with healthy individuals ($P$<0.0001 for all comparisons). A correlation was observed between numbers of IL-1b, IL-8, and IL-17 mRNA expressing PBMC and the degree of neurological impairment as measured by the Scandinavian Stroke Scale 1 to 3 days after onset of symptoms [Kostulas N et al. Increased IL-1beta, IL-8, and IL-17 mRNA expression in blood mononuclear cells observed in a prospective ischemic stroke study. Stroke. 1999 Oct; 30(10):2174-9]. There is no data about the protein level in stroke or in any stroke mimicking condition.

The Fas ligand (FasL) is the ligand of Fas. The Fas system is an apoptosis-signaling receptor located on the cell surface that belongs to the tumor necrosis factor receptor family. The interaction between Fas and its ligand, Fas ligand (FasL) leads to apoptosis. The soluble form of the anti-apoptotic regulator Fas (sFas) and the proapoptotic factors soluble FasL (sFasL) may be measured. Following brain bleedings, sFas, which is thought to inhibit Fas interaction with its ligand, was decreased within the first 24 hours after intracerebral hemorrhage. This fact is in agreement with other studies involving cerebral ischemia, like that reported by Tarkowski et al. [Tarkowski E, et al. Intrathecal expression of proteins regulating apoptosis in acute stroke. Stroke. 1999 Feb;30(2):321-7.]. In their study, sFas level was decreased in cerebrospinal fluid from ischemic stroke patients and was inversely correlated with brain infarct volume and neurologic deficit 3 weeks and 3 months after the event.

In stroke models, Male Sprague-Dawley rats subjected to 2 h middle cerebral artery occlusion with 2 h intraischemic mild hypothermia (33°C) were assayed for Fas, FasL and caspase-8 expression. Ischemia increased Fas, but decreased FasL by approximately 50-60% at 6 and 24 h post-insult. Mild hypothermia significantly reduced expression of Fas and processed caspase-8 both by approximately 50%, but prevented ischemia-induced FasL decreases [Liu L et al. FasL shedding is reduced by hypothermia in experimental stroke. J Neurochem. 2008 May 13].

Reverse transcriptase (RT)-PCR revealed that all glioblastoma cell lines and primary astrocytic brain tumors express FasL. Immunohistochemically, FasL was predominantly expressed on the plasma membrane of glioma cells. These results suggest that FasL expression is common in human astrocytic brain tumors and may cause apoptosis of glioma cells if Fas expression is induced [Gratas C et al. Fas ligand expression in glioblastoma cell lines and primary astrocytic brain tumors. Brain Pathol. 1997 Jul; 7(3):863-9]. However, FasL has not been evaluated in the plasma of any stroke mimicking condition.

b Nerve growth factor (bNGF) is the best characterized neurotrophic factor. Considerable evidence suggests that the nerve growth factor influences the development and maintenance of the basal forebrain cholinergic neurons. However, in the CNS, the trophic action of the nerve growth factor is not limited to the basal forebrain cholinergic neurons. In pathological conditions, the nerve growth factor also plays an important role in neuronal survival and regeneration. For example, the level of nerve growth factor increases in the hippocampus and the cerebral cortex after hypoxic injury [Lorez et al. Nerve growth factor increases in adult rat brain after hypoxic injury. Neurosci. Lett. 98:339-344]. Stanzani et al. [Nerve growth factor and transforming growth factor-beta serum levels in acute stroke patients. Possible involvement of neurotrophins in cerebrovascular disease. Cerebrovasc Dis. 2001; 12(3):240-4] measured nerve growth factor in stroke patients and healthy controls. Although no significant differences between the groups were demonstrated, in stroke patients, serum neurotrophins were significantly associated with clinical and neuroradiological parameters of brain injury in the acute phases of stroke, suggesting that stroke may modulate peripheral neurotrophin levels.

Reynolds et al. [Reynolds MA et al. Early biomarkers of stroke. Clin Chem. 2003 Oct.; 49(10):1733-9] state that "in addition to markers of cellular activation after brain injury, secreted neurotrophic factors such as nerve growth factor and B-type neurotrophic growth factor have been studied in ischemic brain tissue as neuroprotective agents, although studies correlating their plasma concentrations to stroke phenotypes have not been published to date". In that study, two paired monoclonal antibodies were purchased from R&D Systems. Biotinylated goat-anti-BNGF (cat. no. BAF256) was used as the capture antibody, and alkaline phosphatase-conjugated monoclonal anti-BNGF (cat. no. MAB256) was used as the recorder antibody. In that study, only MMP-9 and vWF showed good univariate discrimination between stroke patients and nondiseased controls ($P$<0.0001). However, because they were not uniquely of brain origin and their plasma concentrations could potentially be increased in disease phenotypes other than stroke, authors expanded their stroke panel to other proteins such as bNGF (although in that study the plasma levels of that protein were similar between controls, TIA, ischemic and hemorrhagic stroke patients). Further, Reynolds et al. did not compare stroke patients to stroke mimic subjects.

Insulin Growth Factor Binding Protein 3 (IGFBP3). The insulin-like growth factors (IGF-I and IGF-II) are polypeptide growth factors that mediate anabolic and somatogenic effects of growth hormone and are synthesized and secreted by numerous cell types. IGFs are also present in the brain and are involved in brain development. After experimental hypoxic injury, there is an increased expression of IGFs in regions with neuronal loss. At present six distinct human IGFBPs have been identified that modulate the biological activity of IGFs. In the circulation IGFs are bound mainly to IGFBP-3, the most abundant circulating IGFBP after the neonatal period, whereas in the cerebrospinal fluid concentrations of IGFBP-2 are higher than those of IGFBP-1 or IGFBP-3. Specific changes in the pattern of IGFBP expression occur in the injured brain. IGFBP-3, physiologically expressed in very low amounts in the central nervous system, is markedly induced within the first few days after experimentally induced injury, preferentially in the cortex.

Plasma IGF-I and IGFBP-3 levels were measured sequentially in 20 patients with acute ischemic stroke who were admitted to a Department of Neurology within 24 hours after onset of symptoms. Eight age-matched control patients being treated at the same time in the neurological department because of intervertebral disk protrusion served as controls. The mean plasma levels of IGFBP-3 in the 20 stroke patients were $2.3\pm0.9$ mg/L on day 1; $2.4\pm1.0$ mg/L on day 3; $2.1\pm0.6$ mg/L on day 5; and $2.2\pm1.1$ mg/L on day 10. The values on days 5 and 10 were significantly lower than the control values of $3.2\pm0.7$ and $3.4\pm0.9$ mg/L, respectively ($P<.05$). Among the stroke subgroups (small infarct, SI; moderate infarct, MI; and large infarct, LI), the mean values on days 1, 3, 5, and 10 were $1.8\pm0.4$, $2.0\pm0.6$, $1.9\pm0.7$, and $1.8\pm0.3$ mg/L for patients with LI; $2.1\pm0.4$, $2.2\pm0.6$, $2.9\pm0.2$, and $2.5\pm0.7$ mg/L for those with MI; and $2.8\pm0.8$, $3.1\pm0.3$, $2.7\pm0.9$, and $3.0\pm0.5$ mg/L for those with SI. Again, the values of those patients with LI were the lowest measured, and the difference was statistically significant compared with those of control subjects and those of patients with SI and MI on days 5 and 10 ($P<.05$). However no data about the situation in stroke mimicking conditions was provided, in whom surprisingly, the authors of the instant invention have identified high levels of this protein [Schwab S et al. Plasma insulin-like growth factor I and IGF binding protein 3 levels in patients with acute cerebral ischemic injury. Stroke. 1997 Sep; 28(9):1744-8; Wilczak N et al. Intravenous tissue plasminogen activator in patients with stroke increases the bioavailability of insulin-like growth factor-1. Stroke. 2006 Sep; 37(9):2368-71].

Regarding stroke mimics, no statistical association was detected between glioma and IGF-binding protein-3. Although medium conditioned by meningiomas (and one glioma) also contained Mr 45,000 and 50,000 IGF BPs, similar in size and/or immunological properties to growth hormone-dependent BPs present in normal human serum (IGFBP-3) [Lönn S et al. Glioma risk in relation to serum levels of insulin-like growth factors. Cancer Epidemiol Biomarkers Prev. 2007 Apr; 16(4):844-6; Unterman TG et al. Production of insulin-like growth factor-binding proteins by human central nervous system tumors. Cancer Res. 1991 Jun 1; 51(11):3030-6].

p55 Tumour necrosis factor receptor (TNFR-1) is a cytokine receptor that binds tumor necrosis factors (TNF). Because "TNF" is often used to describe TNF alpha, "TNFR" is often used to describe the receptors that bind to TNF alpha. However, there several other members of this family that bind to the other TNFs. Dziewulska & Mossakowski [Dziewulska D and Mossakowski MJ. Cellular expression of tumor necrosis factor a and its receptors in human ischemic stroke. Clin Neuropathol. 2003 Jan-Feb; 22(1):35-40] evaluated by immunocytochemistry cellular localization and time-dependent expression of tumor necrosis factor a (TNF-alpha) and its receptors p55 (TNFR-1) and p75 (TNFR-2) in human ischemic brains. They observed them in microglia, neurons, astrocytes, macrophages and blood vessels. Since TNF-alpha expression was very intense and prolonged in microglia, it probably constitutes the main cellular source of the cytokine following cerebral ischemia in humans. Constitutive expression of TNF-alpha receptors was observed in neurons and blood vessels while in other cells it was induced by ischemia.

Elneihoum et al. [Elneihoum AM et al. Leukocyte activation detected by increased plasma levels of inflammatory mediators in patients with ischemic cerebrovascular diseases. Stroke. 1996 Oct; 27(10):1734-8], using enzyme-linked immunosorbent assays, measured the plasma levels of soluble TNF receptor protein-1 p55 (sTNFR-1) in 120 patients with acute ischemic cerebrovascular insult [72 with stroke and 48 with transient ischemic attack (TIA)] and in 35 age- and sex-matched healthy subjects. They showed that compared with the control group, plasma sTNFR-1 levels were higher in the stroke group ($P<0.04$). However, those authors or others never compared those levels with other stroke mimicking conditions.

Growth-related oncogene alpha (CrroA), also known as CXCL1, is a potent neutrophil chemoattractant and activator belonging to CXC chemokines family acting mainly through CXCR2 receptors. The role of CXCL1 in bacterial peritonitis, nephrotoxic nephritis, HIV infection and other inflammatory conditions has been shown. Losy et al. [Losy J et al. CXCL1 (GRO-alpha) chemokine in acute ischaemic stroke patients. Folia Neuropathol. 2005;43(2):97-102] aimed to determine CXCL1 levels in CSF and serum of ischaemic stroke patients within 24 h after the onset of the disease and to compare the results with those of a control group. The control group consisted of 15 patients diagnosed with tension headache, who were age and sex-matched with the stroke patients. The serum CXCL1 level in the stroke patients was $86.9\pm25.1$ pg/ml and did not differ significantly from that in the control group, in which the level was $85.5\pm31.8$ pg/ml. However, CSF CXCL1 level in the stroke patients was $65.6\pm22.3$ pg/ml and was significantly higher ($p<0.01$) than that of the control group, in which the level was $43.8\pm2.3$ pg/ml. This biomarker has not been measured in plasma of any other stroke mimics, in which this biomarker might be really relevant.

Zhou et al. [Zhou Y et al. The chemokine GRO-alpha (CXCL1) confers increased tumorigenicity to glioma cells. Carcinogenesis. 2005 Dec; 26(12):2058-68. Epub 2005 Jul 20] found first that resected glioma specimens were strongly immunoreactive for GRO-alpha expression in cells with the morphology of tumor cells.

Consistent with their role in the inflammatory process of MS, both GRO-alpha and CXCR2 were expressed in activated microglia localized on the border of MS lesions [Filipovic R et al. GRO-alpha and CXCR2 in the human fetal brain and multiple sclerosis lesions. Dev Neurosci. 2003 Mar-Aug; 25(2-4):279-90].

The biomarkers described herein (IL-17, FasL, bNGF, IGFBP3, TNFR-1 and GroA) may be used individually, or as part of panels as described hereinafter, and such panels may comprise 1, 2, 3, 4, 5 or 6, of said herein described biomarkers or individual biomarkers. Preferred panels for the diagnosis and/or prognosis of stroke mimicking conditions comprise a plurality of biomarkers independently selected from the group consisting of IL-17, FasL, bNGF, IGFBP3, TNF-1, GroA, and combinations thereof. Optionally, said panels may include, if desired, more biomarkers, for example, other (known or still unknown) biomarkers, different from those provided by the instant invention, for the diagnosis and/or prognosis of stroke mimicking conditions.

For example, panels may incluye the biomarkers combination "A":

FasL, bNGF and IGFBP3;
IL17, IGFBP3 and GroA;
bNGF, IGFBP3 and GroA;
IL17, FasL, bNGF and IGFBP3;
IL17, bNGF, IGFBP3 and GroA;
bNGF, IGFBP3, TNFR-1 and GroA;
FasL, IGFBP3 and GroA;
FasL, IGFBP3, TNFR-1 and GroA;
IL17, bNGF, IGFBP3, TNFR-1 and GroA;
IL17, FasL, IGFBP3 and GroA;
FasL, bNGF, IGFBP3 and TNFR-1;
FasL, bNGF, IGFBP3 and GroA;
IL17, FasL, IGFBP3, TNFR-1 and GroA;
FasL, bNGF, IGFBP3, TNFR-1 and GroA;
IL17, FasL, bNGF, IGFBP3 and TNFR-1;
IL17, FasL, bNGF, IGFBP3 and GroA; or
IL17, FasL, bNGF, IGFBP3, TNFR-1 and GroA.

Alternatively, panels may include the biomarkers combination "B":

IL17 and FasL;
IL17 and GroA;
IL17 and TNF-1;
IL17 and bNGF;
IL17, TNFR-1 and GroA;
IL17, FasL and GroA;
FasL, bNGF and TNFR-1;
IL17, FasL and bNGF;
IL17, FasL and TNFR-1;
IL17, FasL, TNFR-1 and GroA;
FasL, bNGF, TNFR-1 and GroA;
IL17, bNGF and GroA;
IL17, bNGF and TNFR-1;
IL17, FasL, bNGF and TNFR-1;
IL17, FasL, bNGF and GroA;
IL17, bNGF, TNFR-1 and GroA; or
IL17, FasL, bNGF, TNFR-1 and GroA.

A biomarker panel may be analyzed in a number of fashions well known to those of skill in the art. For example, each member of a panel may be compared to a "reference" value, or a value indicating a particular outcome. A particular diagnosis/prognosis may depend upon the comparison of each biomarker to this value. The skilled person in the art will also understand that the biomarkers provided by the instant invention can be used in combination with other biomarkers, e.g., diagnostic biomarkers, differential diagnostic biomarkers, prognostic biomarkers, stroke differentiating markers, etc., in a single assay or device. For example, certain biomarkers in a panel may be commonly used to diagnose the existence of a stroke mimicking condition, others may be used to diagnose the existence of true stroke, and other members of the panel may indicate if a specific mimic subtype has occurred. Thus, in a particular embodiment, the first method of the invention comprises determining the levels of one or more additional stroke mimicking conditions biomarkers and/or the levels of one or more additional stroke biomarkers.

It will be understood that the first method of the invention can be carried out by determining the level of a variable number of the previously mentioned biomarkers in the biological sample of the subject under study. For example, the level of just one of anyone of said biomarkers, or alternatively, the levels of two or more biomarkers, or three or more biomarkers, or four or more biomarkers, or five or more biomarkers, or even a combination of all of the six biomarkers previously mentioned can be used for performing the first method of the invention. The determination of the levels of combinations of the biomarkers may allow greater sensitivity and specificity in diagnosing stroke mimicking conditions, and may allow better differentiation of stroke mimicking conditions from stroke or other eventually related diseases that may have similar or overlapping biomarkers. Further, if desired, the information provided by the analysis of the biomarkers can be used in combination with other clinical variables in order to improve the diagnostic accuracy of the biomarkers provided by the instant invention. In a particular embodiment, said clinical variables are selected between (i) clinical variables which are absent among stroke mimic subjects and are independent predictors of stroke mimicking conditions; and (ii) clinical variables which are present among stroke patients and are independent predictors of stroke. In this sense, the combination of some biomarkers provided by the instant invention with some clinical variables [which are absent among stroke mimic subjects, such as Hypertension, Atrial Fibrillation and Dyslipidemia or any other vascular risk factors that use to be less frequent in stroke mimicking conditions than in real strokes (such as diabetes, older age, smoking tobacco, history of previous cardiovascular disease...)] that are independent predictors of stroke mimicking conditions may achieve sensitivities of 100% and specificities of 100% in the diagnosis/prognosis of stroke mimicking conditions (Figure 4). In a particular embodiment, the invention relates to a combination of 4 biomarkers (2 high and 2 low markers for stroke mimicking conditions that are IGFBP3, TNFR1, IL-17 and bNGF, respectively) and 3 clinical variables which are absent among stroke mimicking subjects (e.g., Hypertension, Atrial fibrillation and Dyslipidemia) that can be used in the differential diagnosis of stroke mimicking conditions versus stroke, said combination achieving a sensitivity of 100% and a specificity of 100%.

A "sample" or "biological sample", as used herein, means biological material isolated from a subject. The biological sample may contain any biological material suitable for detecting the desired biomarker and may comprise cellular and/or noncellular material from the subject. The sample can be isolated from any suitable biological tissue or fluid such as, for example, blood, plasma, serum, urine, tears, saliva or cerebral spinal fluid (CSF). Preferably, the samples used for the determination of the biomarker profile are samples which can be obtained using minimally invasive procedures. In a preferred embodiment, the samples are samples from serum or plasma. The term "subject" relates to an animal including the human being.

The first method of the invention includes the step of determining the levels of a biomarker independently selected from the group consisting of IL-17, FasL, bNGF, IGFBP3, TNFR-1, GroA and combinations thereof, in a sample and comparing said levels to the levels of the same markers in a reference sample.

A "reference sample", as used herein within the context of the first method of the invention, is understood as a stroke-positive sample, i.e., a sample obtained from a subject who suffers, had suffered, or has been diagnosed as suffering from a stroke. The levels in the "reference sample" of a biomarker may be an absolute or relative amount or concentration of the biomarker, a range of amount or concentration of the biomarker, a minimum and/or maximum amount or concentration of the biomarker, a mean amount or concentration of the biomarker, and/or a median amount or concentration of the biomarker; and, in addition, "'reference levels" of combinations of biomarkers may also be ratios of absolute or relative amounts or concentrations of two or more biomarkers with respect to each other. Appropriate positive and negative reference levels of biomarkers for a particular disease state, phenotype, or lack thereof may be determined by measuring levels of desired biomarkers in one or more appropriate subjects, and such reference levels may be tailored to specific populations of subjects (e.g., a reference level may be age-matched so that comparisons may be made between biomarker levels in samples from subjects of a certain age and reference levels for a particular disease state, phenotype, or lack thereof in a certain age group). Such reference levels may also be tailored to specific techniques that are used to measure levels of biomarkers in biological samples (e.g., immunoassays, etc.), where the levels of biomarkers may differ based on the specific technique that is used.

The reference biomarker profile can be generated from one subject or from a population of two or more subjects. The population, for example, may comprise 3, 4, 5, 10, 15, 20, 30, 40, 50 or more subjects. Furthermore, the reference biomarker profile and the individual's (test) biomarker profile that are compared in the methods of the present invention may be generated from the same individual, provided that the test and reference profiles are generated from biological samples taken at different time points and compared to one another. For example, a sample may be obtained from an individual at the start of a study period. A reference biomarker profile taken from that sample may then be compared to biomarker profiles generated from subsequent samples from the same individual. Such a comparison may be used, for example, to determine the status of a stroke mimicking condition in the subject by repeated classifications over time.

A biomarker is considered to be increased in a sample from the subject under study when the levels are increased with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%: by at least 85%, by at least 90%, by at least 95%, by at least 100%, by at least 110%, by at least 120%, by at least 130%, by at least 140% by at least 150%, or more. Similarly, the biomarker is considered to be decreased when its levels are decreased with respect to a reference sample by at least 5%, by at least 10%, by at least 15%, by at least 20%, by at least 25%, by at least 30%, by at least 35%, by at least 40%, by at least 45%. by at least 5094, by at least 55%. by at least 60%, by at least 65%, by at least 70%. by at least 75%, by at least 80%, by at least 85%, by at least 90%, by at least 95%, or by 100% (i.e., absent).

The first method of the invention comprises the step of determining the level of a subject-derived biomarker in a sample obtained from said subject, wherein said biomarker is independently selected from the group consisting of IL-17, FasL, bNGF, IGFBP3, TNFR-1, GroA and combinations thereof. Numerous methods and devices are well known to the skilled person in the art for the detection and analysis of the biomarkers of the instant invention. With regard to polypeptides or proteins in patient test samples, immunoassay devices and methods are often used (see, e.g., U.S. Pat. Nos. 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792). These devices and methods can utilize labeled molecules in various sandwich, competitive, or noncompetitive assay formats, to generate a signal that is related to the presence or amount of an analyte of interest. Additionally, certain methods and devices, such as biosensors and optical immunoassays, may be employed to determine the presence or amount of analytes without the need for a labeled molecule (see, e.g., U.S. Pat. No. 5,631,171; and 5,955,377). One skilled in the art also recognizes that robotic instrumentation including but not limited to Beckman Access, Abbott AxSym, Roche ElecSys, Dade Behring Stratus systems are among the immunoassay analyzers that are capable of performing the immunoassays taught herein.

Preferably the biomarkers are analyzed using an immunoassay, although other methods are well known to those skilled in the art (e.g., the measurement of biomarker RNA levels, for example, biomarker mRNA levels). Thus, in a particular embodiment, the presence and amount of a biomarker can be generally determined using antibodies specific for each biomarker and detecting specific binding. Any suitable immunoassay may be utilized, for example, Western-bolt assays, enzyme-linked immunoassays (ELISA), radioimmunoassays (RIAs), competitive enzymatic immunoassay (EIA), double-antibody sandwich ELISA (DAS-ELISA), competitive binding assays, and the like. Specific immunological binding of the antibody to the biomarker can be detected directly or indirectly. Direct labels include fluorescent or luminescent tags, metals, dyes, radionuclides, and the like, attached to the antibody. Indirect labels include various enzymes well known in the art, such as alkaline phosphatase, horseradish peroxidase and the like.

The term "antibody" as used herein includes monoclonal, hybridoma supernatants, polyclonal and recombinant antibodies; further, said term also includes fragments of antibodies capable of binding to the antigen of interest (i.e., a biomarker of the invention) such as Fv, Fab, Fab' and F(ab')2, single chain Fv (scFv), diabodies, triabodies, tetrabodies, and chimeric antibodies including humanized antibodies.

The use of immobilized antibodies specific for the biomarkers is also contemplated by the present invention. The antibodies could be immobilized onto a variety of solid supports, such as magnetic or chromatographic matrix particles, the surface of an assay place (such as microtiter wells), pieces of a solid substrate material or membrane (such as plastic, nylon, paper), and the like. An assay strip could be prepared by coating the antibody or a plurality of antibodies in an array on solid support. This strip could then be dipped into the test sample and then processed quickly through washes and detection steps to generate a measurable signal, such as a colored spot.

The analysis of a plurality of biomarkers may be carried out separately or simultaneously with one test sample. For separate or sequential assay of markers, suitable apparatuses include clinical laboratory analyzers such as the ElecSys (Roche), the AxSym (Abbott), the Access (Beckman), the ADVIA(R) CENTAUR(R) (Bayer) immunoassay systems, the NICHOLS ADVANTAGE(R) (Nichols Institute) immunoassay system, etc. Preferred apparatuses or protein chips perform simultaneous assays of a plurality of biomarkers on a single surface. Particularly useful physical formats comprise surfaces having a plurality of discrete, adressable locations for the detection of a plurality of different analytes. Such formats include protein microarrays, or "protein chips" (see, e.g., Ng and Ilag, J. Cell Mol. Med. 6: 329-340 (2002)) and certain capillary devices (see, e.g., U.S. Pat. No. 6,019,944). In these embodiments, each discrete surface location may comprise antibodies to immobilize one or more analyte(s) (e.g., a biomarker) for detection at each location. Surfaces may alternatively comprise one or more discrete particles (e.g., microparticles or nanoparticles) immobilized at discrete locations of a surface, where the microparticles comprise antibodies to immobilize one analyte (e.g., a biomarker) for detection.

Several biomarkers may be combined into one test for efficient processing of a multiple of samples. Several technologies, currently available, have taken the ELISA one step further, allowing researchers to simultaneously track several analytes from a single sample measurement. Most multiplexing technologies are variations on the ELISA. They require roughly the same type of blocking, washing, and incubation steps. Differences appear in the substrate the capture antibody is attached to, e.g., a polystyrene plate, a (coated) glass slide, a polymer bead, or even single-stranded RNA. They may differ, too, in the readout, e.g., a change in colour, fluorescence, or light output, resulting from an enzymatic reaction or a change of fluorescence follows laser excitation, and the signal may be detected by film, CCD camera, or photomultiplier tube. Analysis software - the final step - is often pre-packaged with the system, but in many cases can be purchased separately according to the user's needs. Illustrative, non-limitative examples of this technology include the works by Lash et al. [Lash GE et al. Comparison of three multiplex cytokine analysis systems: Luminex, SearchLight and FAST Quant. J Immunol Methods. 2006 Feb 20; 309(1-2):205-8]. In a particular embodiment, each sample can be assayed 2 times using SearchLight Technology and the mean value of both measurements can be used. SearchLight Chemiluminescent are quantitative, plate-based antibody arrays based on traditional ELISA technique and piezoelectric printing technology. SearchLight Arrays are optimized for the quantitative measurement of multiple analytes (proteins) in serum/plasma. Each well of the microplate provided is pre-spotted with antibodies that capture specific analytes in standards and samples added to the plate. After non-bound proteins are washed away, the biotinylated detecting antibodies are added and bind to a second site on the target proteins. Excess detecting antibody is then removed and SA-HRP or SA-DyLight 800 Fluor is added. For chemiluminescent arrays, SuperSignal ELISA Femto Chemiluminescent Substrate (US Patent No. 6,432,662) is added. The enzyme-substrate (HRP-SuperSignal) reaction produces a luminescent signal that is detected with the SearchLight Plus CCD Imaging System. For infrared arrays, signal from the DyLight 800 Fluor can be measured with the Odyssey® or Aerius® Infrared Imaging Systems from LI-COR Biosciences. The amount of signal produced in each spot is proportional to the amount of each specific protein in the original standard or sample.

In addition, one skilled in the art would recognize the value of testing multiple samples (for example, at successive time points) from the same individual. Such testing of serial samples will allow the identification of changes in biomarker levels over time. Increases or decreases in marker levels, as well as the absence of change in biomarker levels, would provide useful information about the disease status that includes, but is not limited to identifying the approximate time from onset of the event, the presence and amount of salvagable tissue, the appropriateness of drug therapies, the effectiveness of various therapies as indicated by reperfusion or resolution of symptoms, identification of the severity of the event, identification of the disease severity, and identification of the patient's outcome, including risk of future events.

A panel consisting of the markers referenced above may be constructed to provide relevant information related to differential diagnosis. Such a panel may be constructed using 1, 2, 3, 4, 5, 6, or more or individual biomarkers. The analysis of a single biomarker or subsets of biomarkers comprising a larger panel of biomarkers could be carried out by one skilled in the art to optimize clinical sensitivity or specificity in various clinical settings. These include, but are not limited to ambulatory, urgent care, critical care, intensive care, monitoring unit, inpatient, outpatient, physician office, medical clinic, and health screening settings. Furthermore, one skilled in the art can use a single biomarker or a subset of biomarkers comprising a larger panel of biomarkers in combination with an adjustment of the diagnostic threshold in each of the aforementioned settings to optimize clinical sensitivity and specificity. The clinical sensitivity of an assay is defined as the percentage of those with the disease that the assay correctly predicts, and the specificity of an assay is defined as the percentage of those without the disease that the assay correctly predicts (Tietz Textbook of Clinical Chemistry, 2nd edition, Carl Burtis and Edward Ashwood eds., W. B. Saunders and Company, p. 496).

The analysis of biomarkers could be carried out in a variety of physical formats as well. For example, the use of microtiter plates or automation could be used to facilitate the processing of large numbers of test samples. Alternatively, single sample formats could be developed to facilitate immediate treatment and diagnosis in a timely fashion, for example, in ambulatory transport or emergency room settings.

There are commercially available antibodies which recognize and bind to the biomarkers provided by the instant invention (IL-17, FasL, bNGF, IGFBP3, TNFR-1 and GroA). Illustrative, non limitative examples of commercially available antibodies to the biomarkers of the invention include:

IL-17: IL-17 (1) Antibody from Santa Cruz Biotechnology, Inc., reference/catalog number sc-53937; Human IL-17 (Interleukin-17, IL17) ELISA from eBioscience, reference/catalog number 88-7176-22; Human IL-17F DuoSet from R&D Systems reference/catalog number DY1335 DY1335; IL17, Human ELISA Kit from Cell Sciences, reference/catalog number CKH157; Human IL-17 ELISA Set from Thermo Scientific, reference/catalog number ESS0030 or other available antibodies;

FasL: Fas Ligand (CD178; TNFSF6) from R&D Systems, reference/catalog number DFL00; Fas Ligand from Calbiochem reference/catalog number QIA27-1EA; sFas Ligand from Bender MedSystems reference/catalog number BMS260/2TENCE; or other available antibodies;

bNGF: Beta-nerve Growth Factor (NGFB) ELISA from Lifespan Biosciences, reference/catalog number LS-C37648; Human beta-NGF DuoSet from R&D Systems, reference/catalog number DY256; Human NGF Beta Construction Kit from Antigenix America Inc., reference/catalog number RHF280CK; or other available antibodies;

IGFBP3: Human IGFBP3 ELISA Kit from Cell Sciences, reference/catalog number CKH150; Anti-Human IGFBP3 Monoclonal Antibody, Unconjugated, Clone 84728.111, Clone 7D9, Clone 83.8F9 from ABR-Affinity Bioreagents, now sold as Thermo Scientific, references/catalog numbers MA1-20185, MA1-82910, MA1-84011; Anti-Human IGFBP3 Monoclonal Antibody, Unconjugated, Clone YY07 from Santa Cruz Biotechnology, Inc., reference/catalog number sc-74124; or other available antibodies;

TNFR1: Human sTNF-R (60kDa) ELISA Kit from Bender MedSystems, reference/catalog number BMS203TENCE;

Anti-Human TNFRSF1A Polyclonal Antibody, from Abnova, reference/catalog number H00007132-A01; or other available antibodies; and

**GroA:** GROα (129-3) Antibody from Santa Cruz Biotechnology, Inc., reference/catalog number sc-74071; GRO alpha (CXCL1) from R&D Systems, reference/catalog number DGR00; or other available antibodies.

Alternatively, the generation and selection of said antibodies may be accomplished by several ways. For example, one way is to purify the proteins or polypeptides of interest (i.e., the biomarkers provided by the instant invention) or to synthesize them using, e.g., solid phase peptide synthesis methods well known in the art [see, e.g., Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996]. The selected biomarkers may then be injected, for example, into mice or rabbits, to generate polyclonal or monoclonal antibodies. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures [Antibody Engineering: A Practical Approach (Borrebaeck, C., ed.), 1995, Oxford University Press, Oxford; J. Immunol. 149, 3914-3920 (1992)].

In addition, numerous publications have reported the use of phage display technology to produce and screen libraries of polypeptides for binding to a selected target (see, e.g, Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698). A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means (see, e.g., U.S. Pat. No. 6,057,098).

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

Those skilled in the art will recognize that many approaches can be taken in producing antibodies or binding fragments and screening and selecting for affinity and specificity for the various polypeptides, but these approaches do not change the scope of the invention.

Alternatively, in a particular embodiment, the presence and amount of a biomarker of the invention can be determined by quantifying the expression level of the corresponding gene encoding said biomarker (IL-17, FasL, bNGF, IGFBP3, TNFR-1 and GroA). The expression level of a gene can be quantified by quantifying the level of the mRNA encoding said biomarker, or alternatively, the level of the complementary DNA (cDNA) to said mRNA. In this case, the method shall include an extraction step to obtain total RNA, which can be performed by means of conventional techniques (Chomczynski et al., Anal. Biochem., 1987, 162: 156; Chomczynski P., Biotechniques, 1993, 15:532). Any conventional method can be used within the framework of the invention to detect and quantify the mRNA levels encoding the biomarkers of the invention or their corresponding cDNA. By way of non-limiting example, the mRNA levels encoding said biomarkers can be quantified by conventional methods, for example, methods comprising mRNA amplification and the quantification of the product of said mRNA, such as electrophesis and staining, or alternatively, by means of Southern blot together with suitable probes, Northern blot together with specific probes for the mRNA of the corresponding genes or of their corresponding cDNA, mapping with S1 nuclease, RT-LCR, hybridization, microarrays, etc., for example, by real time quantitative PCR using a suitable marker. Similarly, the levels of cDNA corresponding to said mRNA encoding the biomarkers of the invention can also be quantified using conventional techniques; in this case, the method includes a synthesis step of the corresponding cDNA by means of reverse transcription (RT) of the corresponding mRNA followed by amplification and quantification of the amplification product of said cDNA; in a particular embodiment, the amplification is carried out qualitatively or quantitatively via PCR, using oligonucleotide primers which specifically amplify regions of the genes encoding the biomarkers of the invention.

## II. Diagnosis of stroke

As it has been previously mentioned, the inventors of the instant invention have identified some biomarkers present in the serum or plasma of subjects (patients) suffering from stroke which are present at different levels with respect to the serum/plasma of subjects who exhibit one or more stroke-associated symptoms but that have not suffered from a stroke (these subjects are generically referred to herein as "stroke mimic subjects" and the condition they suffer is generically referred to herein as a "stroke mimicking condition"). These biomarkers can then be used, for example, in a rapid, sensitive and specific diagnostic assay to be used in the diagnosis and differentiation of stroke from stroke mimic subjects.

Thus, in an aspect, the invention relates to a method (hereinafter "the second method of the invention") for diagnosing stroke in a subject, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the subject is diagnosed as having stroke when the levels of one or more of biomarkers selected from IL-17, FasL, bNGF and combinations thereof, is/are increased with respect to the level of the same biomarker in a reference sample and/or the levels of one or more biomarkers selected from IGFBP3, TNFR1, GroA and combinations thereof is/are decreased with respect to the level of the same biomarker in a reference sample, wherein said reference sample is a stroke mimicking condition-positive sample.

The different aspects of the second method of the invention (the methods used for the determination of the levels of the markers, the nature of the sample which is to be studied, and the thresholds for consideration of a marker as having been increased or decreased) are essentially as defined previously in respect of the first method of the invention.

Thus, the second method of the invention, as the first method of the invention, may essentially consist of the aforementioned steps or may include further steps, and it is to be understood that the method, in a preferred embodiment, is a method carried out *in vitro,* i.e. not practiced on the human or animal body.

The terms "stroke", "stroke mimic subject" and "stroke mimicking conditions", as used herein, have been previously defined.

Similarly, the term "biomarker", as used herein, has been previously defined. According to the second method of the invention, suitable biomarkers for the diagnosis of stroke include IL-17, FasL, bNGF, IGFBP3, TNFR-1 and GroA, whose characteristics have been previously mentioned.

A "reference sample", as used herein within the context of the second method of the invention, is understood as a stroke mimicking condition-positive sample, i.e., a sample obtained from a subject who has not been diagnosed of stroke but who exhibits one symptom or a constellation of symptoms that mimic those symptoms exhibited by subjects who have been diagnosed of stroke (i.e., stroke mimic subject). Stroke-associated symptoms, stroke mimic subjects and stroke mimicking conditions have been previously defined. In a preferred embodiment, the reference sample is obtained from a subject who exhibits one or more of the stroke-associated symptoms but that has not suffered from a stroke (i.e., a stroke mimic subject).

The biomarkers described herein (IL-17, FasL, bNGF, IGFBP3, TNFR-1 and GroA) may be used individually, or as part of panels as described hereinafter, and such panels may comprise 1, 2, 3, 4, 5 or, 6, of said herein described biomarkers or individual biomarkers. Preferred panels for the diagnosis and/or prognosis of stroke comprise a plurality of biomarkers independently selected from the group consisting of IL-17, FasL, bNGF, IGFBP3, TNFR-1, GroA, and combinations thereof Illustrative, non-limitative examples of panels which can be used include those combinations of biomarkers previously identifed as combinations A and B. Optionally, said panels may include, if desired, more biomarkers, for example, other (known or still unknown) biomarkers, different from those provided by the instant invention, for the diagnosis and/or prognosis of stroke. Thus, the biomarkers provided by the instant invention can be used in combination with additional biomarkers, e.g., diagnostic biomarkers, differential diagnostic biomarkers, prognostic biomarkers, stroke differentiating markers, etc. For example, certain biomarkers in a panel may be commonly used to diagnose the existence of stroke, others may be used to diagnose stroke mimicking conditions, and other members of the panel may indicate if a specific mimic subtype has occurred. Thus, in a particular embodiment, the second method of the invention comprises determining the levels of one or more additional stroke biomarkers and/or the levels of one or more additional stroke mimicking conditions biomarkers.

Similarly to the first method of the invention, it will be understood that the second method of the invention can be carried out by determining the level of a variable number of the previously mentioned biomarkers in the biological sample of the subject under study. For example, the level of just one of anyone of said biomarkers, or alternatively, the levels of two or more biomarkers, or three or more biomarkers, or four or more biomarkers, or five or more biomarkers, or even a combination of all of the six biomarkers previously mentioned can be used for performing the second method of the invention. The determination of the levels of combinations of the biomarkers may allow greater sensitivity and specificity in diagnosing stroke and may allow better differentiation of stroke from stroke mimicking conditions or other eventually related diseases that may have similar or overlapping biomarkers. Further, if desired, the information provided by the analysis of the biomarkers can be used in combination with other clinical variables in order to improve the diagnostic accuracy of the biomarkers provided by the instant invention. In a particular embodiment, said clinical variables are selected between (i) clinical variables which are absent among stroke mimic subjects and are independent predictors of stroke mimicking conditions; and (ii) clinical variables which are present among stroke patients and are independent predictors of stroke. In this sense, the combination of some biomarkers provided by the instant invention with some clinical variables (which are absent among stroke mimicking subjects) that are independent predictors of stroke [such as Hypertension, Atrial Fibrillation and Dyslipidemia or any other vascular risk factors that use to be less frequent in stroke mimicking conditions than in real strokes (such as diabetes, older age, smoking tobacco, history of previous cardiovascular disease...)] may achieve sensitivities of 100% and specificities of 100% in the diagnosis/prognosis of stroke mimicking conditions (Figure 4). In a particular embodiment, the invention relates to a combination of 4 biomarkers (IL-17, bNGF, IGFBP3 and TNFR-1) and 3 clinical variables which are absent among stroke patients (Hypertension, Atrial Fibrillation and Dyslipidemia) that can be used in the differential diagnosis of stroke versus stroke mimicking conditions, said combination achieving a sensitivity of 100% and a specificity of 100%.

A "reference sample", as used herein within the context of the second method of the invention, is a stroke-positive sample; i.e., a sample obtained from a subject who suffers, had suffered, or has been diagnosed as suffering from a stroke. As mentioned above, the thresholds for consideration of a marker as having been increased or decreased) are essentially as defined previously in respect of the first method of the invention.

### III. Method for the determination of the efficacy of a therapy for a stroke mimicking condition

The invention also provides a method for the determination of the efficacy of the therapy for a stroke mimicking condition. Thus, in another aspect, the invention relates to a method (hereinafter "the third method of the invention") for the determination of the efficacy of a therapy for a stroke mimicking condition in a subject, said subject having been diagnosed of a stroke mimicking condition and having been treated with said therapy, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the therapy is considered as effective for the treatment of a stroke mimicking condition when the level(s) of one or more of said biomarkers reaches/reach normal levels.

The different aspects of the third method of the invention (the methods used for the determination of the levels of the markers, the nature of the sample which is to be studied, the thresholds for consideration of a marker as having been increased or decreased) are essentially as defined previously in respect of the first or second method of the invention.

As used herein, the expression "the therapy is considered effective when the level(s) of one or more of said biomarkers reaches/reach normal levels" within the scope of the third method of the invention means that if the altered (high or low) levels of the biomarkers in a pathological state (i.e., in a subject suffering from a stroke mimicking condition), after administration of said therapy to the subject in need thereof, are "normalized", i.e., fall within the normal levels for said biomarkers (i.e., the level of said biomarkers in healthy subjects) then said therapy is suitable for treating said stroke mimicking condition in said subject. Normal levels of a biomarker can be easily determined by the skilled person in the art by conventional means, for example, by determining the level of said biomarker in a population of healthy subjects. By illustrative, if the level of a particular biomarker is higher in a subject suffering from a stroke mimicking condition than in healthy subjects and the level of said biomarker is lowered until normal levels after administration of a specific drug, then said therapy is potentially useful for treating said stroke mimicking condition in said subject. Similarly, if the level of a particular biomarker is lower in a subject suffering from a stroke mimicking condition than in healthy subjects and the level of said biomarker is raised until normal levels after administration of a specific drug, then said therapy is potentially useful for treating said stroke mimicking condition in said subject.

In order to determine the efficacy of a therapy for a stroke mimicking condition in a subject, the level(s) of the biomarker(s) is/are determined in a sample derived from the subject wherein the efficacy of the therapy is to be tested.

**IV. Method for the determination of the efficacy of a therapy for stroke**

The invention also provides a method for the determination of the efficacy of the therapy for stroke. Thus, in another aspect, the invention relates to a method (hereinafter "the fourth method of the invention") for the determination of the efficacy of a therapy for stroke in a subject, said subject having been diagnosed of stroke and having been treated with said therapy, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the therapy is considered as effective for the treatment of stroke when the level(s) of one or more of said biomarkers reaches/reach normal levels. The different aspects of the fourth method of the invention (the methods used for the determination of the levels of the markers, the nature of the sample which is to be studied, the thresholds for consideration of a marker as having been increased or decreased) are essentially as defined previously in respect of the first or second method of the invention.

As used herein, the expression "the therapy is considered effective when the level(s) of one or more of said biomarkers reaches/reach normal levels" within the scope of the fourth method of the invention means that if the altered (high or low) levels of the biomarkers in a pathological state (i.e., in a subject suffering from stroke), after administration of said therapy to the subject in need thereof, are "normalized", i.e., fall within the normal levels for said biomarkers (i.e., the level of said biomarkers in healthy subjects) then said therapy is suitable for treating stroke in said subject. Normal levels of a biomarker can be easily determined by the skilled person in the art by conventional means, for example, by determining the level of said biomarker in a population of healthy subjects.

By illustrative, if the level of a particular biomarker is higher in a subject suffering from stroke than in healthy subjects and the level of said biomarker is lowered until normal levels after administration of a specific drug, then said therapy is potentially useful for treating stroke in said subject. Similarly, if the level of a particular biomarker is lower in a subject suffering from stroke than in healthy subjects and the level of said biomarker is raised until normal levels after administration of a specific drug, then said therapy is potentially useful for treating stroke in said subject. In order to determine the efficacy of a therapy for stroke in a subject, the level(s) of the biomarker(s) is/are determined in a sample derived from the subject wherein the efficacy of the therapy is to be tested.

**V. Method for the identification of compounds suitable for the treatment of stroke or for the treatment of stroke mimicking conditions**

The authors of the present invention have also developed methods for the identification of compounds suitable for the treatment of stroke or stroke mimicking conditions. The identification of a series of biomarkers whose levels are increased or decreased with respect to reference samples, allows the screening for compounds in a model of stroke or stroke mimicking conditions which are capable of restoring the levels of the markers to those found in said reference samples.

Thus, in another aspect, the invention relates to a method (hereinafter "the fifth method of the invention") for the identification of compounds suitable for the treatment of stroke which comprises determining in a biological sample of a patient suffering from stroke and having been treated with a candidate compound the level(s) of one or more biomarkers independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the compound is considered as effective for the treatment of stroke when the level(s) of one or more of said biomarkers reaches/reach normal levels after administration of said compound to a reference sample.

The different aspects of the fifth method of the invention (the methods used for the determination of the levels of the markers, the nature of the sample which is to be studied, the thresholds for consideration of a marker as having been increased or decreased) are essentially as defined previously in respect of the first method of the invention.

The term "reference sample", as used in respect of the fifth method of the invention, relates to a stroke-positive sample or a sample containing expression systems (e.g., native or recombinant cells or recombinant expression systems) which express a compound selected from IL-17, FasL, bNGF, IGFBP3, TNFR1, GroA and combinations thereof.

As used herein, the expression "the compound is considered effective for the treatment of stroke when the level of one or more of said biomarkers reaches/reach normal levels" within the scope of the fifth method of the invention means that if the altered (high or low) levels of the biomarkers usually present in a pathological state (e.g., stroke), after administration of said compound to the reference sample, are "normalized", i.e., fall within the normal levels for said biomarkers (i.e., the level of said biomarkers in healthy subjects) then said compound is potentially useful as a candidate for treating stroke. Normal levels of a biomarker can be easily determined by the skilled person in the art by conventional means, for example, by determining the level of said biomarker in a population of healthy subjects.

By illustrative, if the level of a particular biomarker is higher in a reference sample (stroke-positive sample) than in healthy subjects and the level of said biomarker is lowered until normal levels after administration of a specific compound, then said compound is potentially useful for treating stroke. Similarly, if the level of a particular biomarker is lower in a reference sample (stroke-positive sample) than in healthy subjects and the level of said biomarker is raised until normal levels after administration of a specific compound, then said compound is potentially useful for treating stroke.

Further, in another aspect, the invention relates to a method (hereinafter "the sixth method of the invention") for the identification of compounds suitable for the treatment of stroke mimicking conditions which comprises determining in a biological sample of a patient suffering from stroke and having been treated with a candidate compound the level (s) of one or more biomarkers independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the compound is considered as effective for the treatment of a stroke mimicking condition when the level(s) of one or more of said biomarkers reaches/reach normal levels after administration of said compound to a reference sample.

The different aspects of the sixth method of the invention (the methods used for the determination of the levels of the markers, the nature of the sample which is to be studied, the thresholds for consideration of a marker as having been increased or decreased) are essentially as defined previously in respect of the first method of the invention.

The term "reference sample", as used in respect of the sixth method of the invention, relates to a stroke mimicking condition-positive sample or a sample containing expression systems (e.g., native or recombinant cells or recombinant expression systems) which express a compound selected from IL-17, FasL, bNGF, IGFBP3, TNFR1, GroA and combinations thereof.

As used herein, the expression "the compound is considered effective for the treatment of a stroke mimicking condition when the level of one or more of said biomarkers reaches/reach normal levels" within the scope of the sixth method of the invention means that if the altered (high or low) levels of the biomarkers usually present in a pathological state (e.g., a stroke mimicking condition), after administration of said compound to the reference sample, are "normalized", i.e., fall within the normal levels for said biomarkers (i.e., the level of said biomarkers in healthy subjects) then said compound is potentially useful as a candidate for treating stroke. Normal levels of a biomarker can be easily determined by the skilled person in the art by conventional means, for example, by determining the level of said biomarker in a population of healthy subjects. By illustrative, if the level of a particular biomarker is higher in a reference sample (stroke mimicking condition-positive sample) than in healthy subjects and the level of said biomarker is lowered until normal levels after administration of a specific compound, then said compound is potentially useful for treating a stroke mimicking condition. Similarly, if the level of a particular biomarker is lower in a reference sample (stroke mimicking condition-positive sample) than in healthy subjects and the level of said biomarker is raised until normal levels after administration of a specific compound, then said compound is potentially useful for treating a stroke mimicking condition.

In a particular embodiment, the invention provides *in vitro* methods for identifying agents (e.g., candidate compounds or test compounds) that are capable of modulating (increasing or decreasing) the expression of a biomarker selected from the group consisting of IL-17, FasL, bNGF, IGFBP3, TNFR1, GroA and combinations thereof in a suitable expression system. Said expression system may be a native cell or a recombinant expression system (e.g., a recombinant cell) which expresses a compound selected from the group consisting of IL-17, FasL, bNGF, IGFBP3, TNFR1, GroA and combinations thereof In another particular embodiment, the invention provides *in vivo* methods for identifying agents that are capable of modulating the expression of a biomarker selected from the group consisting of IL-17, FasL, bNGF, IGFBP3, TNFR1, GroA and combinations thereof in a non-human transgenic animal model. Agents identified through the screening methods of the invention (i.e., the fifth and sixth methods of the invention) are potential therapeutics for use in treating a subject in need thereof.

Examples of suitable animals for use in the screening method of the invention include, but are not limited to, mice, rats, rabbits, monkeys, guinea pigs, dogs and cats. In accordance with this embodiment, the test compound or a control compound is administered (e.g., orally, rectally or parenterally such as intraperitoneally or intravenously) to a suitable animal and the effect on the levels of one or more of the biomarkers provided by this invention (IL-17, FasL, bNGF, IGFBP3, TNFR1 and GroA) are determined. Examples of agents include, but are not limited to, nucleic acids (e.g., DNA and RNA), carbohydrates, lipids, proteins, peptides, peptidomimetics, small molecules and other drugs. Agents can be obtained using any of the numerous approaches in combinatorial library methods known in the art. Test compounds further include, for example, antibodies (e.g., polyclonal, monoclonal, humanized, anti-idiotypic, chimeric, and single chain antibodies as well as Fab, F(ab') 2, Fab expression library fragments, and epitope-binding fragments of antibodies). Further, agents or libraries of compounds may be presented, for example, in solution, on beads, chips, bacteria, spores, plasmids or phage. If the compound is a low-molecular weight compound, then this can be generated by various methods known to the art, preferably synthetically, in particular by combinatorial chemistry, or by biochemical methods, in particular by recombinant recombination or purification from biological probes. The compound is of low molecular weight ("small molecules") or the library is composed of molecules with low molecular weight ("small molecule library"). A "small molecule library" is defined as a complex collection of compounds, which are produced in a nonbiological way, that means which are not produced by recombinant expression, like for instance most protein or peptide libraries.

"Small molecules" can be generated by various methods known to the art, but are preferably produced by synthetically, more preferably by combinatoric chemistry, to generate a compound library with a maximum chemical diversity within the constraints of predicted attractive drug characteristics. If the compound to be assayed for its suitability for the treatment of stroke or a stroke mimicking condition is a peptide or a protein, then it can be generated by various methods known to the art for their use as candidate compounds, but they are preferably produced by biochemical methods, more preferably by recombinant expression in prokaryotic or eukaryotic cells.

The compound to be tested for its suitability for the therapy of stroke or a stroke mimicking condition can be formulated with a pharmaceutically acceptable carrier to produce a pharmaceutical composition, which can be administered to a subject. A pharmaceutically-acceptable carrier can be, for example, water, sodium phosphate buffer, phosphate-buffered saline, normal saline or Ringer's solution or other physiologically-buffered saline, or other solvent or vehicle such as a glycol, glycerol, an oil such as olive oil or an injectable organic ester. A pharmaceutically acceptable carrier can also contain physiologically acceptable compounds that act, for example, to stabilize or increase the absorption of the modulatory compound. One skilled in the art would know that the choice of a pharmaceutically acceptable carrier, including a physiologically acceptable compound, depends, for example, on the route of administration of the composition.

**VI. Method for the differential diagnosis of stroke**

The authors of the present invention have also identified a series of biomarkers whose levels are altered in patients suffering stroke with respect to patients suffering a stroke mimicking condition. Said biomarkers can then be used, for example, in a rapid, sensitive and specific diagnostic assay to be used in the diagnosis and differentiation of stroke from stroke mimickinmg conditions.

Thus, in another aspect, the invention relates to a method (hereinafter "the seventh method of the invention") for the differential diagnosis of stroke or stroke mimicking conditions in a subject which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, and comparing the levels of said biomarkers with the levels of the same markers in a stroke-positive sample and in a stroke mimicking condition-positive sample wherein

(i) the subject is diagnosed as having stroke when the levels of one or more of biomarkers selected from IL-17, FasL, bNGF and combinations thereof, is/are increased with respect to the level of the same biomarker in a stroke mimicking condition-positive sample and/or the levels of one or more biomarkers selected from IGFBP3, TNFR1, GroA and combinations thereof is/are decreased with respect to the level of the same biomarker in a stroke mimicking condition-positive sample, and

(ii) the subject is diagnosed as having a stroke mimicking condition when the levels of one or more of biomarkers selected from IL-17, FasL, bNGF and combinations thereof, is/are decreased with respect to the level of the same biomarker in a stroke-positive sample and/or the levels of one or more biomarkers selected from IGFBP3, TNFR1, GroA and combinations thereof is/are increased with respect to the level of the same biomarker in a stroke-positive sample.

The method is carried out using essentially the same methods as described for the first and second method of the invention regarding the technology used for the detection and quantification of the levels of the biomarkers. In fact, the different aspects of the seventh method of the invention (the methods used for the determination of the levels of the markers, the nature of the sample which is to be studied, the thresholds for consideration of a marker as having been increased or decreased) are essentially as defined previously in respect of the first method of the invention.

This method can be used for the rapid, sensitive and specific diagnosis and differentiation of stroke from stroke mimicking conditions. The appropriate treatment for stroke or stroke mimicking conditions is highly desirable for the reasons mentioned in the Background of the Invention. Thus, once a diagnosis is obtained, the clinician can readily select a treatment regimen that is compatible with the diagnosis. For example, some Health Authorities (e.g., U.S. Food and Drug Administration) have approved the use of clot-dissolving drug tissue plasminogen activator (tPA) to treat ischemic stroke, which constitutes 70-80 percent of all strokes. tPA is effective only if given promptly; therefore, for maximum benefit, the therapy must be started within 3 hours of the onset of stroke symptoms, making rapid diagnosis and differentiation of stroke critical.

This need for speed in stroke evaluation is critical because early treatment (within hours of stroke onset) is the single most critical factor likely to improve outcome with modem treatments. Accordingly, the present invention provides methods of early differential diagnosis to allow for appropriate intervention in acute time windows (i.e., when tPA should be administered for ischemic stroke). Invention methods can further be combined with CT scan(s), wherein a CT scan can be used to rule out hemorrhagic stroke, and invention methods can be used to diagnose and differentiate other types of stroke.

## VII. Kit for the diagnosis of stroke or stroke mimicking conditions

The invention also relates to kits for the analysis of the biomarkers provided by the present invention (IL-17, FasL, bNGF, IGFBP3, TNFR1 and GroA). Thus, in a further aspect, the invention relates to a kit comprising a reagent or a set of reagents for the analysis of a biomarker selected from the group consisting of IL-17, FasL, bNGF, IGFBP3, TNFR-1, GroA and combinations thereof. Such a kit preferably comprises devices and reagents for the analysis of at least one of said biomarkers, preferably, 2, 3, 4, 5 or even said 6 biomarkers, and, optionally, instructions for performing the assay. Such kits preferably contain sufficient reagents to perform one or more such determinations.

In a particular embodiment, said kits contain an antibody which recognizes and binds to a biomarker provided by the present invention (IL-17, FasL, bNGF, IGFBP3, TNFR1 and GroA); preferably, said kits contain 1, 2, 3, 4, 5 or even 6 antibodies, each one capable of recognizing and binding to a specific biomarker provided by the present invention. Optionally, the kits may contain one or more means for using information obtained from immunoassays performed for a marker panel to rule in or out certain diagnoses.

These kits can be used for diagnosing and/or prognosing stroke or stroke mimicking conditions. Therefore, in another aspect, the invention relates to the use of said kits in the diagnosis and/or prognosis of stroke or a stroke mimicking conditions.

## EXAMPLE 1

### Identification of biomarkers capable to differentiate true stroke from stroke mimicking conditions

Patients with an acute stroke suspicion admitted at the emergency department of a Universitary Hospital have been prospectively studied. The identification of biomarkers capable to differentiate true stroke from stroke mimics is very important due to the high rate of stroke mimics which are not properly identified. This rate depends on the type of medical Service and Hospital where a patient with a suspition of stroke arrives, ranging from 5% at the Stroke Units up to 50% in the Community Hospitals or ambulances. Therefore, this study was carried out in order to identify biomarkers capable to differentiate true stroke from stroke mimics.

### 1. Materials and methods

#### 1.1 Study population

Study population was recruited in Vall d'Hebron Hospital (Barcelona - Spain). In front of a stroke suspicion (less than 24 hours from symptoms onset) the study protocol was initiated. In short, blood samples were obtained at patient arrival to the emergency department and later on a complete diagnostic protocol allowed to decide the final diagnosis of "true stroke" (stroke) or a "stroke mimicking condition" (stroke mimic).

Among those patients who had had an acute ischemic stroke admitted within the first 3 hours after symptom onset, a standard protocol to decide if they were candidates to receive thrombolytic treatments was performed. Said patients underwent urgent carotid ultrasound and transcranial Doppler (TCD) examinations and those patients that had a documented Middle Cerebral Artery occlusion on TCD were selected for treatment with t-PA in a standard 0.9 mg/kg dose (10% bolus, 90% continuous infusion during 1 hour).

A population of 230 cases was analysed in the present study. Of them, 146 were stroke patients, 61 mimics and 23 healthy controls. Subjects of these three study groups were randomly selected from our plasma library in order to simulate the mimics rate of interest for the study (around 30%, equivalent to the mimic rates of most referral centers).

#### 1.2 Clinical Protocol

1.2.1 Demographic data

Demographic data and a detailed history of vascular risk factors were obtained from each patient. Table 1 shows main demographic data in both groups of stroke and mimics.

**Table 1**

| Demographics and risk factors profile | | | | |
|---|---|---|---|---|
| Variable | All Patients | Stroke | MIMIC | P-value |
| Age | $70.1 \pm 14.7$ (18-98) | $71.7 \pm 13.2$ | $66.1 \pm 17.2$ | 0.025 |
| Sex (females) | 95 (46.1%) | 47.30% | 43.30% | 0.607 |
| Tobacco | 31 (16.7%) | 20.60% | 6% | 0.018 |
| Hypertension | 95 (48.7% | 58.60% | 20% | <0.001 |
| Diabetes Mellitus | 36 (18.4%) | 20.50% | 12% | 0.178 |
| Atrial Fibrillation | 55 (28.2%) | 36.60% | 4% | <0.001 |
| Coronary Disease | 27 (13.8%) | 17.10% | 4% | 0.02 |
| Dyslipidemia | 49 (25.3%) | 30.60% | 10% | 0.004 |
| Previous stroke | 37 (18.9%) | 17.10% | 24% | 0.284 |

1.2.2 Clinical examination

Clinical examination was performed on admission and serially at follow up. Stroke severity was assessed by using the National Institutes of Health Stroke Scale (NIHSS).

1.2.3 Diagnostic work-up

On admission, all patients underwent a Computed Tomography (CT) of the brain in order to identify any disease responsible of the stroke symptoms, if any. CT was repeated after 24-48 hours (or earlier when rapid neurological deterioration occurred) to evaluate the final lesion. If CT was inconclusive for making a final diagnosis, patients received cranial Magnetic Resonance Imaging (MRI).

In order to identify potential mechanism of cerebral infarction, a set of diagnostic tests was performed that included electrocardiogram, chest radiography, carotid ultrasonography, complete blood count and leukocyte differential and blood biochemistry in all patients; when indicated, some patients also underwent special coagulation tests, transthoracic echocardiography and Holter monitoring. With this information, and the neuroimaging data, previously defined etiologic subgroups were determined [TOAST classification]. All this data also allowed an accurate classification of stroke mimicking conditions.

1.2.4 Informed consents

Studies for identifying stroke biomarkers useful for aiding in diagnosis have been approved by the Ethics Committee of the Vall d'Hebron Hospital and all patients or relatives gave informed consent.

1.3 Blood samples

Peripheral blood samples were drawn from each patient at study entry (before t-PA administration, if recommended) within 3 hours from stroke onset. EDTA tubes were used to collect the blood. Plasma was immediately separated by centrifugation at 3,000 rpm for 15 minutes and stored at -80°C. Assays were performed according to the manufacturer's instructions.

1.4 Immunoassays

1.4.1 Assay development

SearchLight Chemiluminescent are quantitative, plate-based antibody arrays based on traditional ELISA technique and piezoelectric printing technology. SearchLight Arrays have been optimized for the quantitative measurement of multiple analytes (proteins) in serum; EDTA, heparin, and sodium citrate plasma; culture supernatants; and other sample types.

Each sample was assayed in duplicate using the SearchLight Technology and the mean value of both measurements was used. Each well of the microplate provided was pre-spotted with antibodies that capture specific analytes in standards and samples added to the plate. After non-bound proteins were washed away, the biotinylated detecting antibodies were added and bound to a second site on the target proteins. Excess detecting antibody was then removed and SuperSignal ELISA Femto Chemiluminescent Substrate (US Patent 6,432,662) was added. The enzyme-substrate (HRP-SuperSignal) reaction produced a luminescent signal that was detected with the SearchLight Plus CCD Imaging System. The amount of signal produced in each spot is proportional to the amount of each specific protein in the original standard or sample. ArrayVision™ is the software used to quantify the images. It offers more that 8 curves for each molecule and automatically adjusts the results. The mean and standar deviations are automatically calculated and the data can be saved as a Microsoft Excel file, permitting the validation of results obtained. The method has been previously used in our laboratory with excellent results [Rosell A, et al. A matrix metalloproteinase protein array reveals a strong relation between MMP-9 and MMP-13 with diffusion-weighted image lesion increase in human stroke. Stroke. 2005 Jul; 36(7):1415-20].

1.4.2 SearchLight Custom Arrays

Custom arrays allow to measure the particular proteins which are most relevant (showing up-regulation or down-regulation) in a disease model. After screening protein libraries (more than 150 proteins assayed, as shown in Table 2), in a group of selected and very well phenotyped stroke patients, mimics and healthy controls, 23 key biomarkers were selected and combined in arrays in which a much larger number of patients was tested. All the antibodies and necessary products for screening those biomarkers are supplied by Pierce (Thermo Fisher Scientific). In this study, inventors designed several custom arrays covering 23 proteins of interest [IL-17, MPIF-1, IL-2RG, CD14, BetaNGF, FasL, IL-16, IL-18, IL-1RA, IFN-gamma, IgE, G-CSF, GM-CSF, GroA, Leptin, HGH, IP-10, IGFBP3, IGFBP1, E-cadherin, PDGFbb, TNFR1 and RANTES], that showed significance or trends to distinguish stroke from stroke mimicking conditions, and that were tested in all 230 included subjects in order to replicate those findings.

**Table 2 Library of proteins used for the initial screening of biomarkers useful to differentiate stroke mimicking conditions from real strokes**

| Angiogenesis Factors | Cytokines | Neurotrophic Factors |
|---|---|---|
| Ang-2 | GM-CSF | β-NGF |
| FGF basic | G-CSF | BDNF |
| HB-EGF | IFNα | CNTF |
| HGF | IFNγ | GDNF |
| KGF | IL-1α | NT3 |
| NGAL | IL-1β | |
| PDGF-AA | IL-1ra | **Other Biomarkers** |
| PDGF-AB | IL-2 | α2-Macroglobulin |
| PDGF-BB | IL-3 | A-SAA |
| TIMP-1 | IL-4 | Acrp-30 (Adiponectin) |
| TIMP-2 | IL-5 | AR (Amphiregulin) |
| TPO | IL-6 | Apo A-1 |
| VEGF | IL-7 | Apo B-100 |
| VEGF-C | IL-8 | β-sAPP |
| VEGF-D | IL-9 | Cathepsin-D |
| VEGF-R1 | IL-10 | C-peptide |
| VEGF-R2 | IL-11 | CD14 |
| | IL-12p40 (p40/homodimer) | CD30 (TNFRSF8) |
| **Cell Adhesion Molecules** | IL-12p70 (heterodimer) | CD40L |
| E-Cadherin | IL-13 | CGα |
| E-Selectin | IL-15 | Clusterin |
| ICAM-1 | IL-16 | COX-2 |
| L-Selectin | IL-17 | CRP (C-reactive protein) |
| P-Selectin | IL-18 | D-Dimer |
| VCAM-1 | IL-23 | ER (Epiregulin) |
| | M-CSF | ErbB2 |

(continued)

| Angiogenesis Factors | Cytokines | Neurotrophic Factors |
|---|---|---|
| **Chemokines** | TNFα | FasL |
| ENA-78 | TNFα Trimer Speci.c | Fibrinogen |
| Eotaxin | | Fibronectin |
| Eotaxin-2 | **Cytokine Receptor** | IGFBP-1 |
| Exodus-2 | IL-1 RII | IGFBP-3 |
| GROα | IL-2R | Inhibin A |
| GROγ | IL-2Rγ | Leptin |
| HCC-4 (CCL-16) | IL-4R | LIF |
| I-309 | IL-6R | MPO (Myeloperoxidase) |
| IP-10 | TNF-R1 | MRP 8/14 |
| ITAC | TNF-RII | NGAL |
| Lymphotactin | | NT-proBNP |
| MCP-1 | **Growth Factors** | OPG (Osteoprotegrin) |
| MCP-2 | EGF | OPN (Osteopontin) |
| MCP-3 | EGFR | PAI-1 Active |
| MCP-4 | HGH | PAI-1 Total |
| MDC | TGFα | PAPP-A |
| MIF | TGFβ1 | PEDF |
| MIG | TGFβ2 | PLGF (Placental Growth Factor) |
| MIP-1α | | Prolactin |
| MIP-1β | **Immunoglobulins** | Protein C |
| MIP-1δ | IgE | RAGE |
| MIP-3α | | RANK |
| MIP-3β | | RANKL |
| MIP-4 (PARC) | **Matrix Metalloproteinases** | Resistin |
| MPIF-1 | MMP-1 | SHBG |
| NAP-2 | MMP-2 | SP-C |
| RANTES | MMP-3 | SP-D |
| RBP4 | MMP-7 | Thrombomodulin |
| SDF-1β | MMP-8 | Tissue Factor |
| TARC | MMP-9 | TRAIL |
| | MMP-10 | TSP-1 (Thrombospondin-1) |
| | MMP-13 | TSP-2 (Thrombospondin-2) |
| | | TWEAK |
| | | Visfatin |
| | | VonWillebrand Factor |

1.5 Statistical Analyses

Descriptive and frequency statistical analysis were obtained and comparisons were made by use of the SPSS statistical package, version 15.0. Statistical significance for intergroup differences was assessed by the Fisher's exact test for categorical variables and the Student's $t$ test and ANOVA for continuous variables. Whether biomarker values were normally distributed was tested (Kolmogorov-Smirnov and P-P plot will be used). To assess variations during the temporal profile of selected biomarkers a Friedman test was performed, although baseline biomarker levels were used for following analyses. When indicated, Mann-Whitney $U$ and Pearson tests were used. To calculate the sensitivity and specificity for biomarkers values to predict stroke/mimic diagnosis, a receiver operator characteristic (ROC) curve was configured for each individual biomarker. Sensitivity, specificity, possitive predictive value (PPV) and negative predictive values (NPV) were calculated. A logistic regression analysis was performed to determine factors (including clinical variables and biomarkers) that might be considered independent predictors of stroke/mimic diagnosis. A $p$ value <0.05 was considered statistically significant. Models of stroke versus mimic's prediction were generated.

## 2. Results

From a screening in a large (more than 150) protein-antibody library, it has been possible to identify several biomarkers useful to differentiate stroke from mimics.

Patients from the inventors' database were selected in order to simulate usual mimics rate from the healthcare system level inventors were interested (i.e. around 30% in this study, which is the usual mimic's rate in comarcal hospitals that refer stroke patients to large centers where they may be treated). Therefore, the following data refer to a population of 230 cases; of them, 146 were stroke patients, 61 mimics and 23 healthy controls in which the most interesting 23 biomarkers coming from that previous screening had been analyzed in depth.

Mimics identified in the inventors' population corresponded to: seizures, tumors, migraine, hypoglycemia, syncope, peripheral nervous system diseases, toxics, and non neurological diseases. In all those cases the neurologist on call thought to be in front of a stroke patient. Table 1 shows main demographic data in both groups of stroke and mimics.

Units for all shown biomarkers in this application are picogram per milliliter (pg/mL).

### 2.1 Selected biomarkers for differentiating stroke from mimics

Several biomarkers were significantly different between real stroke and stroke mimicking conditions (mimic). Among them the most significant were: IL-17, bNGF, IL2RG, IGFBP3, TNFR1, GroA, PDGFBB and FasL. Figure 1 shows the distribution of some of those biomarkers (IL-17, IL2RG, FasL, bNGF, GroA, IGFBP3, TNFR1, and PDGFBB) among different studied groups of subjects (healthy controls, stroke patients and stroke mimics conditions).

After selecting the best cut-off level for each biomarker looking for a compromise between better sensitivity offering acceptable specificity we selected the best biomarkers. Those were six biomarkers strong enough as for being independent predictors as it could be seen later in the multivariate analysis. Three of those markers were significantly (p<0.05) elevated in stroke patients as compared to mimics (IL-17, bNGF and FasL) and three of those markers were lower among stroke patients as compared with mimics (IGFBP3, TNFR1 and GroA).

Most of those selected biomarkers were not influenced by risk factors as shown in Table 3.

**Table 3**
**Modifications in most relevant biomarkers related with vascular risk factors**

| Risk factors | | TNFR-1 | IGFBP-3 | bNGF | FASL | GROA |
|---|---|---|---|---|---|---|
| Age | R | 0.18 | ........ | ........ | ........ | ........ |
| | p | 0.01 | ........ | ........ | ........ | ........ |
| Sex | Male | ........ | ........ | ........ | ........ | ........ |
| | Female | ........ | ........ | ........ | ........ | ........ |
| | p | ........ | ........ | ........ | ........ | ........ |
| Tobacco | No | ......... | 1091840 (744983-1621404) | ........ | ........ | ........ |
| | Yes | ......... | 863084 (484807-1267816) | ........ | ........ | ........ |
| | p | ......... | 0.019 | ........ | ........ | ........ |
| Hypertension | No | ........ | ........ | 0.9 (0.5-1.3) | ........ | ........ |
| | Yes | ........ | ........ | 1.2 (0.7-1.6) | ........ | ........ |
| | p | ........ | ........ | 0.007 | ........ | ........ |
| Atrial Fibrillation | No | ........ | ........ | ........ | 11.6 (3.7-29.1) | 250.7 (152.8-437) |
| | Yes | ........ | ........ | ........ | 19.2 (10.4-33.3) | 168.5 (77.7-280.6) |

(continued)

**Modifications in most relevant biomarkers related with vascular risk factors**

| Risk factors | | TNFR-1 | IGFBP-3 | bNGF | FASL | GROA |
|---|---|---|---|---|---|---|
| | *p* | ........ | ........ | ........ | *0.011* | *0.001* |
| Dyslipidemia | No | ........ | ........ | 0.9 (0.5-1.3) | ........ | ........ |
| | Yes | ........ | ........ | 1.2 (0.9-1.6) | ........ | ........ |
| | *p* | ........ | ........ | *0.012* | ........ | ........ |

In fact, after multivariate analysis as shown in Table 4, those six markers remained independent predictors of stroke.

**Table 4**
**Multivariate analysis, showing independent predictors of stroke versus mimics**

| Clinical Factors | Odds Ratio (I.C. 95%) | P-value |
|---|---|---|
| Hypertension | 3.711 (1.554-8.862) | 0.003 |
| Atrial Fibrillation | 19.661 (4.367-88.524) | <0.001 |
| Dyslipidemia | 3.946 (1.303-11.946) | 0.015 |
| Tobacco | 7.586 (2.041-28.192) | 0.002 |
| **Biomarkers** | **Odds Ratio (I.C. 95%)** | **P-value** |
| IL17 >0.05 | 31.833 (2.297-441.175) | 0.01 |
| FASL >13.8 | 2.847 (1.287-6.298) | 0.01 |
| BNGF>1.25 | 7.843 (2.80-21.973) | <0.001 |
| IGFBP3 <2165872.35 | 8.954 (1.649-48.629) | 0.011 |
| TNFR1 <3271 | 3.250 (1.474-7.168) | 0.003 |
| GROA <424.6 | 2.619 (1.023-6.705) | 0.045 |
| **Clinical Factors + Biomarkers** | **Odds Ratio (I.C. 95%)** | **P-value** |
| IL17 >0.05 | 114.766 (3.496-3767.611 | 0.008 |
| BNGF >1.25 | 9.193 (2.264-37.323) | 0.002 |
| IGFBP3 <2165872.35 | 34.552 (2.613-456.953) | 0.007 |
| TNFR1 <3291 | 8.699 (2.877-26.299) | <0.001 |
| Hypertension | 3.467 (1.125-10.687) | 0.03 |
| Atrial Fibrillation | 26.72 (4.766-149.805) | <0.001 |
| Dyslipidemia | 8.116 (1.795-36.697) | 0.007 |

There were also several clinical variables such as hypertension, atrial fibrillation, dyslipidemia and smokers; that were also independent predictors of stroke versus mimics. The final model integrating all clinical and biomarkers data is also shown.

Figure 2 represents ROC curves for those models of independent predictors of stroke patients versus stroke mimic subjects. Three ROC curves were constructed comparing diagnostic accuracy of selected biomarkers (IL-17, FasL, bNGF, GroA, IGFBP3 and TNFR1), selected clinical variables (Hypertension, Atrial Fibrillation, Dyslipidemia or Smoking Tobacco) or the combination of both biomarkers and clinical variables (IL-17, bNGF, IGFBP3, TNFR1, Hypertension, Atrial Fibrillation and Dyslipidemia). Although the biomarkers model had better AUC = 0.856 than the clinical model, with an AUC = 0.833, it was the combination of both biomarkers plus clinical variables, the model that offered the best AUC = 0.933, adding significant information (p<0.05) to both clinical data alone and biomarkers alone.

2.2 Sensitivity and specificity of the panel

A model with 6 biomarkers, IL-17, FasL, bNGF, GroA, IGFBP3 and TNFR1, (i.e., 3 high and 3 low biomarkers for stroke and 3 high and 3 low biomarkers for stroke mimicking conditions) that were independent predictors of stroke versus stroke mimicking conditions achieved sensitivities of 100% and specificities of 100% as shown in Figure 3. These sensitivities and specificities were even improved when combining biomarkers with clinical data as shown in Figure 4. On the one hand, Figure shows the results of a model with 4 biomarkers (2 high and 2 low markers for stroke, that are IL-17, bNGF, IGFBP3, and TNFR1 respectively) and 3 clinical variables (present among stroke patients, that are Hypertension, Atrial Fibrillation and Dyslipidemia) that are independent predictors of stroke versus stroke mimicking conditions; some combinations achieving sensitivities of 100% and specificities of 100%; and, on the other hand, Figure 4 shows the results of a model with 4 biomarkers (2 high and 2 low markers for mimics that are IGFBP3, TNFR1, IL-17 and bNGF, respectively) and 3 clinical variables (absent among stroke mimic subjects that are Hypertension, Atrial Fibrillation and Dyslipidemia) that are independent predictors of stroke mimicking conditions versus stroke; some combinations achieving sensitivities of 100% and specificities of 100%.

Table 5 shows that GroA (91.7%) was the individual marker with the best sensitivity and Table 6 that IL17 is the one with best specificity (98.4%). Interestingly, the negative predictive value for stroke obtained by different combinations of three biomarkers was 100%. Moreover, four different combinations of only two biomarkers had 100% positive predictive value.

**Table 5**

**Combinations among biomarkers offering the best sensitivity to differentiate stroke versus mimics**

| Combination | Sensitivity | Specificity | NPV (% MIMIC) |
|---|---|---|---|
| C234 | 100.0% | 15.0% | 100.0% |
| C146 | 100.0% | 13.3% | 100.0% |
| C346 | 100.0% | 13.3% | 100.0% |
| C1234 | 100.0% | 13.3% | 100.0% |
| C1346 | 100.0% | 11.7% | 100.0% |
| C1456 | 100.0% | 11.7% | 100.0% |
| C3456 | 100.0% | 11.7% | 100.0% |
| C246 | 100.0% | 10.0% | 100.0% |
| C2456 | 100.0% | 10.0% | 100.0% |
| C13456 | 100.0% | 10.0% | 100.0% |
| C1246 | 100.0% | 8.3% | 100.0% |
| C2345 | 100.0% | 8.3% | 100.0% |
| C2346 | 100.0% | 8.3% | 100.0% |
| C12456 | 100.0% | 8.3% | 100.0% |
| C23456 | 100.0% | 8.3% | 100,0% |
| C12345 | 100.0% | 6.7% | 100.0% |
| C12346 | 100.0% | 6.7% | 100.0% |
| C123456 | 100.0% | 6.7% | 100.0% |
| C46 | 99.3% | 15.0% | 90.0% |
| C456 | 99.3% | 13.3% | 88.9% |
| C1345 | 99.3% | 13.3% | 88.9% |
| C134 | 98.6% | 23.3% | 87.5% |
| C245 | 99.3% | 10.0% | 85.7% |

(continued)

**Combinations among biomarkers offering the best sensitivity to differentiate stroke versus mimics**

| Combination | Sensitivity | Specificity | NPV (% MIMIC) |
|---|---|---|---|
| C356 | 97.9% | 26.7% | 84.2% |
| C34 | 97.9% | 25.0% | 83.3% |
| C1356 | 97.9% | 25.0% | 83.3% |
| C1256 | 98.6% | 16.7% | 83.3% |
| C2356 | 98.6% | 16.7% | 83.3% |
| C1245 | 99.3% | 8.3% | 83.3% |
| C145 | 98.6% | 15.0% | 81.8% |
| C345 | 98.6% | 15.0% | 81.8% |
| C12356 | 98.6% | 15.0% | 81.8% |
| C256 | 97.9% | 18.3% | 78.6% |
| C36 | 96.6% | 30.0% | 78.3% |
| C136 | 96.6% | 28.3% | 77.3% |
| C156 | 96.6% | 28.3% | 77.3% |
| C24 | 97.9% | 16.7% | 76.9% |
| C124 | 97.9% | 15.0% | 75.0% |
| C235 | 94.5% | 38.3% | 74.2% |
| C1235 | 94.5% | 36.7% | 73.3% |
| C236 | 97.2% | 18.3% | 73.3% |
| C14 | 95.9% | 25.0% | 71.4% |
| C1236 | 97.2% | 16.7% | 71.4% |
| C56 | 94.5% | 30.0% | 69.2% |
| C126 | 96.6% | 18.3% | 68.8% |
| C16 | 93.8% | 31.7% | 67.9% |
| C26 | 95.9% | 20.0% | 66.7% |
| C45 | 96.6% | 16.7% | 66.7% |
| C125 | 90.3% | 40.0% | 63.2% |
| (6) GroA<424.6 | 91.7% | 33.3% | 62.5% |
| C135 | 85.5% | 55.0% | 61.1% |
| C25 | 89.0% | 41.7% | 61.0% |
| (4) IGFBP-3<2165872.35 | 92.4% | 26.7% | 59.3% |
| C123 | 82.2% | 57.4% | 57.4% |
| C35 | 82.1% | 56.7% | 56.7% |
| C23 | 78.8% | 59.0% | 53.7% |
| C15 | 77.9% | 61.7% | 53.6% |
| PDFGBB<2716.1 | 81.5% | 46.7% | 50.9% |
| C13 | 56.8% | 88.5% | 46.2% |
| (5)TNFR-1<3231.65 | 69.0% | 63.3% | 45.8% |

(continued)

**Combinations among biomarkers offering the best sensitivity to differentiate stroke versus mimics**

| Combination | Sensitivity | Specificity | NPV (% MIMIC) |
|---|---|---|---|
| CD14<5346130.75 | 84.8% | 27.9% | 44.7% |
| MPIF1>441.3 | 80.8% | 32.8% | 41.7% |
| (3) bNGF>1.25 | 45.2% | 90.2% | 40.7% |
| C12 | 60.3% | 62.3% | 39.6% |
| IL2RG>2.65 | 38.4% | 95.1% | 39.2% |
| IGBP-1<23403.35 | 64.8% | 51.7% | 37.8% |
| (2) FasL>13.8 | 54.8% | 63.9% | 37.1% |
| IGE<54442.2 | 49.0% | 70.0% | 36.2% |
| (1) IL17>0.05 | 22.6% | 98.4% | 34.7% |

wherein "C" followed of one or more figures indicates the combination of specific biomarkers, wherein: "1" is IL17, "2" is FasL, "3" is bNGF, "4" is IGFBP3, "5" is TNFR-1 and "6" is GroA.

**Table 6**

**Combinations among biomarkers offering the best specificity to differentiate stroke versus mimics**

| Combination | Sensitivity | Specificity | PPV (% ICTUS) |
|---|---|---|---|
| C12 | 17.1% | 100.0% | 100.0% |
| C15 | 13.1% | 100.0% | 100.0% |
| C14 | 12.4% | 100.0% | 100.0% |
| C13 | 11.0% | 100.0% | 100.0% |
| C145 | 11.0% | 100.0% | 100.0% |
| C125 | 9.0% | 100.0% | 100.0% |
| C234 | 9.0% | 100.0% | 100.0% |
| C123 | 8.9% | 100.0% | 100.0% |
| C124 | 8.3% | 100.0% | 100.0% |
| C1245 | 7.6% | 100.0% | 100.0% |
| C2345 | 7.6% | 100.0% | 100.0% |
| C135 | 5.5% | 100.0% | 100.0% |
| C134 | 4.8% | 100.0% | 100.0% |
| C1234 | 4.1% | 100.0% | 100.0% |
| C1235 | 4.1% | 100.0% | 100.0% |
| C1345 | 4.1% | 100.0% | 100.0% |
| C12345 | 3.4% | 100.0% | 100.0% |
| C34 | 24.1% | 98.3% | 97.2% |
| (1) IL17>0.05 | 22.6% | 98.4% | 97.1% |
| C345 | 17.9% | 98.3% | 96.3% |
| IL2RG>2.65 | 38.4% | 95.1% | 94.9% |
| C245 | 24.1% | 96.7% | 94.6% |

(continued)

**Combinations among biomarkers offering the best specificity to differentiate stroke versus mimics**

| Combination | Sensitivity | Specificity | PPV (% ICTUS) |
|---|---|---|---|
| C235 | 11.0% | 98.3% | 94.1% |
| C35 | 26.2% | 95.0% | 92.7% |
| C25 | 32.4% | 93.3% | 92.2% |
| (3) bNGF>1.25 | 45.2% | 90.2% | 91.7% |
| C23 | 21.2% | 95.1% | 91.2% |
| C45 | 44.8% | 86.7% | 89.0% |
| C24 | 26.9% | 91.7% | 88.6% |
| IGBP-3<795163 | 41.4% | 86.7% | 88.2% |
| PDFGBB<1301.1 | 30.1% | 90.0% | 88.0% |
| (4) TNFR-1<2638.4 | 55.9% | 78.3% | 86.2% |
| (5) GroA<210.75 | 62.8% | 73.3% | 85.0% |
| IGE<54442.2 | 49.0% | 70.0% | 79.8% |
| (2) FasL>13.8 | 54.8% | 63.9% | 78.4% |
| IGBP-1<23403.35 | 64.8% | 51.7% | 76.5% |
| MPIF1>441.3 | 80.8% | 32.8% | 74.2% |
| CD14<5346130.75 | 84.8% | 27.9% | 72.7% |

wherein "C" followed of one or more figures indicate the combination of specific biomarkers, wherein: "1" is IL17, "2" is FasL, "3" is bNGF, "4" is TNFR-1 and "5" is GroA.

Figure 5 shows how several clinical variables and biomarkers, selected in the multivariate analysis (IL-17, bNGF, IGFBP3, TNFR1, Hypertension, Atrial Fibrillation and Dyslipidemia) allowed building up a model with a great predictive value to screen stroke patients. In fact, this model may screen perfectly a population with no error (misdiagnosis of stroke) but correctly identifying 10.2% of mimics that might remain in the small hospitals. If it is admitted an error rate in stroke diagnosis (not ruled-out properly) as low as 1.4% the method of the invention will allow to correctly allocate mimics and avoid urgent transfers to large hospitals in as many as 46.9% of mimics that will be correctly identified.

In individual patients, and again coming from the model including both risk factors + biomarkers, the below formula may be applied in order to obtain the estimated probability of stroke:

$$1/(1+e^{13.68-4.74*IL17>0.05-2.22*BNGF>1.25-3.54*IGFBP3<2165872.35-2.16*TNFR1<3271.05-1.24*HTA-3.28*FA-2.09*DL})$$

## 3. Discussion

### 3.1 Identified biomarkers

Results of this extensive study show that screening a large protein-antibody library is a feasible strategy to search for stroke mimicking condition biomarkers. This strategy allowed inventors to identify biomarkers able to differentiate stroke from mimics. The biomarkers identified in this study can be used, alone or in combination with other biomarkers, to differentiate stroke mimicking conditions from stroke.

### 3.2 Diagnostic level achieved by the panel

The implementation of the identified model will allow to guide stroke management and to decide which patients should urgently be derived to large centers where advanced therapies (i.v or i.a. thrombolysis, etc.) may be conducted. This model may screen perfectly a population with no error (misdiagnosis of stroke) but correctly identifying 10.2% of mimics that might remain in the small hospitals. If it is admitted an error rate in stroke diagnosis as low as 1.4% the method of the invention will allow correctly allocate mimics and avoid urgent transfers to large hospitals in almost 50% of the cases. This means a huge safe of costs for the healthcare systems.

**Claims**

1. A method for diagnosing a stroke mimicking condition in a subject, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the subject is diagnosed as having a stroke mimicking condition when the levels of one or more of biomarkers selected from IL-17, FasL, bNGF and combinations thereof, is/are decreased with respect to the level of the same biomarker in a reference sample and/or the levels of one or more biomarkers selected from IGFBP3, TNFR1, GroA and combinations thereof is/are increased with respect to the level of the same biomarker in a reference sample, wherein said reference sample is a stroke-positive sample.

2. A method for diagnosing stroke in a subject, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the subject is diagnosed as having stroke when the levels of one or more of biomarkers selected from IL-17, FasL, bNGF and combinations thereof, is/are increased with respect to the level of the same biomarker in a reference sample and/or the levels of one or more biomarkers selected from IGFBP3, TNFR1, GroA and combinations thereof is/are decreased with respect to the level of the same biomarker in a reference sample, wherein said reference sample is a stroke mimicking condition-positive sample.

3. Method according to claim 1 or 2, which comprises determining the levels of 1, 2, 3, 4, 5 or 6, of said biomarkers.

4. Method according to claim 3, which comprises determining the levels of the following combination of biomarkers:

   FasL, bNGF and IGFBP3;
   IL17, IGFBP3 and GroA;
   bNGF, IGFBP3 and GroA;
   IL17, FasL, bNGF and IGFBP3;
   IL17, bNGF, IGFBP3 and GroA;
   bNGF, IGFBP3, TNFR-1 and GroA;
   FasL, IGFBP3 and GroA;
   FasL, IGFBP3, TNFR-1 and GroA;
   IL17, bNGF, IGFBP3, TNFR-1 and GroA;
   IL17, FasL, IGFBP3 and GroA;
   FasL, bNGF, IGFBP3 and TNFR-1;
   FasL, bNGF, IGFBP3 and GroA;
   IL17, FasL, IGFBP3, TNFR-1 and GroA;
   FasL, bNGF, IGFBP3, TNFR-1 and GroA;
   IL17, FasL, bNGF, IGFBP3 and TNFR-1;
   IL17, FasL, bNGF, IGFBP3 and GroA;
   IL17, FasL, bNGF, IGFBP3, TNFR-1 and GroA;
   IL17 and FasL;
   IL17 and GroA;
   IL17 and TNFR-1;
   IL17 and bNGF;
   IL17, TNFR-1 and GroA;
   IL17, FasL and GroA;
   FasL, bNGF and TNFR-1;
   IL17, FasL and bNGF;

IL17, FasL and TNFR-1;
IL17, FasL, TNFR-1 and GroA;
FasL, bNGF, TNFR-1 and GroA;
IL17, bNGF and GroA;
IL17, bNGF and TNFR-1;
IL17, FasL, bNGF and TNFR-1;
IL17, FasL, bNGF and GroA;
IL17, bNGF, TNFR-1 and GroA; or
IL17, FasL, bNGF, TNFR-1 and GroA.

5. Method according to anyone of claims 1 to 4, which further comprises determining the levels of one or more additional stroke mimicking conditions biomarkers and/or the levels of one or more additional stroke biomarkers.

6. Method according to anyone of claims 1 to 5, which further comprises combining the results of the biomarker(s) determination with some clinical variables, said clinical variables being selected between (i) clinical variables which are absent among stroke mimic subjects and are independent predictors of stroke mimicking conditions; and (ii) clinical variables which are present among stroke patients and are independent predictors of stroke.

7. Method according to claim 6, which comprises determining the level of biomarkers IL-17, bNGF, IGFBP3 and TNFR-1 and combining the results thereof with 3 clinical variables, wherein said clinical variables are Hypertension, Atrial fibrillation and Dyslipidemia.

8. A method for the determination of the efficacy of a therapy for a stroke mimicking condition in a subject, said subject having been diagnosed of a stroke mimicking condition and having been treated with said therapy, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the therapy is considered as effective for the treatment of a stroke mimicking condition when the level(s) of one or more of said biomarkers reaches/reach normal levels.

9. A method for the determination of the efficacy of a therapy for stroke in a subject, said subject having been diagnosed of stroke and having been treated with said therapy, which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the therapy is considered as effective for the treatment of stroke when the level(s) of one or more of said biomarkers reaches/reach normal levels.

10. A method for the identification of compounds suitable for the treatment of stroke which comprises determining in a biological sample of a patient suffering from stroke and having been treated with a candidate compound the level(s) of one or more biomarkers independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the compound is considered as effective for the treatment of stroke when the level(s) of one or more of said biomarkers reaches/reach normal levels after administration of said compound to a reference sample.

11. A method for the identification of compounds suitable for the treatment of stroke mimicking conditions which comprises determining in a biological sample of a patient suffering from stroke and having been treated with a candidate compound the level(s) of one or more biomarkers independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, wherein the compound is considered as effective for the treatment of a stroke mimicking condition when the level(s) of one or more of said biomarkers reaches/reach normal levels after administration of said compound to a reference sample.

12. Method according to claim 10 or 11, wherein said reference sample is selected from the group consisting of a stroke-positive sample, a stroke mimicking condition-positive sample and a sample containing an expression system which express a compound selected from IL-17, FasL, bNGF, IGFBP3, TNFR1, GroA and combinations thereof.

**13.** A method for the differential diagnosis of stroke or stroke mimicking conditions in a subject which comprises determining the level of a subject-derived biomarker in a sample obtained from said subject, wherein said biomarker is independently selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof, and comparing the levels of said biomarkers with the levels of the same markers in a stroke-positive sample and in a stroke mimicking condition-positive sample wherein

(i) the subject is diagnosed as having stroke when the levels of one or more of biomarkers selected from IL-17, FasL, bNGF and combinations thereof, is/are increased with respect to the level of the same biomarker in a stroke mimicking condition-positive sample and/or the levels of one or more biomarkers selected from IGFBP3, TNFR1, GroA and combinations thereof is/are decreased with respect to the level of the same biomarker in a stroke mimicking condition-positive sample, and

(ii) the subject is diagnosed as having a stroke mimicking condition when the levels of one or more of biomarkers selected from IL-17, FasL, bNGF and combinations thereof, is/are decreased with respect to the level of the same biomarker in a stroke-positive sample and/or the levels of one or more biomarkers selected from IGFBP3, TNFR1, GroA and combinations thereof is/are increased with respect to the level of the same biomarker in a stroke-positive sample.

**14.** A kit comprising a reagent or a set of reagents for the analysis of a biomarker selected from the group consisting of interleukin-17 (IL-17), Fas ligand (FasL), b nerve growth factor (bNGF), insulin growth factor binding protein 3 (IGFBP3), p55 tumour necrosis factor receptor (TNFR-1), growth-related oncogene alpha (GroA) and combinations thereof.

**15.** Use of a kit according to claim 10, in the diagnosis and/or prognosis of stroke or a stroke mimicking condition.

**Distribution of relevant identified biomarkers in healthy controls, stroke patients and stroke mimicking conditions**

**Figure 1**

**Figure 1(cont.)**

**Figure 1(cont.)**

**Figure 1(cont.)**

ROC curves for independent predictors of stroke versusmimics

Figure 2

EP 2 166 358 A1

Predictive value of determined combinations of the 6 identified biomarkersfor stroke or mimics diagnosis

Figure 3

**Predictive value of determined combinations ofthe 4 identified biomarkers together with the 3 identified clinical variables (vascular risk factors) for stroke or mimics diagnosis**

Figure 4

EP 2 166 358 A1

Percentage ofmimics or strokes  identified by each of the combinations of biomarkers and clinical dataselected in our model

Figure 5

EP 2 166 358 A1

Europäisches
Patentamt

European
Patent Office

Office européen
des brevets

**EUROPEAN SEARCH REPORT**

Application Number

EP 08 16 4495

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/199000 A1 (VALKIRS GUNARS E [US] ET AL) 23 October 2003 (2003-10-23) relevant for non unity | 1 | INV. G01N33/68 |
| Y | * claims 1-3,11,19,23-26,36 * | 1-13,15 | |
| X | WO 2004/059293 A (BIOSITE INC [US]; BUECHLER KENNETH F [US]; MAISEL ALAN [US]; ANDERBERG) 15 July 2004 (2004-07-15) relevant for non unity * page 25, paragraph 73 - paragraph 74 * | 1 | |
| Y | * claims 78,79,85,86,103124,133,136,138-140 * * page 126, paragraph 366 * | 1-13,15 | |
| X | US 6 197 525 B1 (YAO ZHENGBIN [CN] ET AL) 6 March 2001 (2001-03-06) * claims 1-3 * | 14 | |
| X | YOO JEONGMI ET AL: "Monoclonal antibodies reactive with chicken interleukin-17" VETERINARY IMMUNOLOGY AND IMMUNOPATHOLOGY, vol. 121, no. 3-4, February 2008 (2008-02), pages 359-363, XP002503762 ISSN: 0165-2427 * abstract * | 14 | TECHNICAL FIELDS SEARCHED (IPC) G01N |
| Y | KOSTULAS N ET AL: "Increased IL-1beta, IL-8, and IL-17 mRNA expression in blood mononuclear cells observed in a prospective ischemic stroke study." STROKE; A JOURNAL OF CEREBRAL CIRCULATION OCT 1999, vol. 30, no. 10, October 1999 (1999-10), pages 2174-2179, XP002503763 ISSN: 0039-2499 * abstract * | 1-13,15 | |

-/--

The present search report has been drawn up for all claims

| Place of search | Date of completion of the search | Examiner |
|---|---|---|
| The Hague | 27 March 2009 | van der Kooij, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt
European
Patent Office
Office européen
des brevets

# EUROPEAN SEARCH REPORT

Application Number

EP 08 16 4495

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 946 255 B1 (KAYAGAKI NOBUHIKO [JP] ET AL) 20 September 2005 (2005-09-20) * claims 1-6,10 * | 14 | |
| A | LUKASZEWSKI R A ET AL: "Presymptomatic prediction of sepsis in intensive care unit patients." CLINICAL AND VACCINE IMMUNOLOGY : CVI JUL 2008, vol. 15, no. 7, July 2008 (2008-07), pages 1089-1094, XP002520425 ISSN: 1556-679X * abstract * | 1-15 | |
| X | US 2004/219509 A1 (VALKIRS GUNARS E [US] ET AL) 4 November 2004 (2004-11-04) * claims 1,2,9,10,29 * * page 2, paragraph 13 - paragraph 16 * * page 8, paragraph 73 * | 8-12 | |
| A | DE FREITAS IVEL ET AL: "Serum levels of the apoptosis-associated molecules, tumor necrosis factor-alpha/tumor necrosis factor type-I receptor and Fas/FasL, in sepsis." CHEST JUN 2004, vol. 125, no. 6, June 2004 (2004-06), pages 2238-2246, XP002520414 ISSN: 0012-3692 * abstract * | 1-15 | TECHNICAL FIELDS SEARCHED (IPC) |
| X | US 2004/237124 A1 (PONS JAUME [US] ET AL) 25 November 2004 (2004-11-25) * claims 1,6,8,10,17,19,21,27,32 * | 14 | |

-/--

The present search report has been drawn up for all claims

| Place of search | Date of completion of the search | Examiner |
|---|---|---|
| The Hague | 27 March 2009 | van der Kooij, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 08 16 4495

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RIIKONEN R S ET AL: "Cerebrospinal nerve growth factor--a marker of asphyxia?" PEDIATRIC NEUROLOGY FEB 1999, vol. 20, no. 2, February 1999 (1999-02), pages 137-141, XP008104158 ISSN: 0887-8994 * abstract * * figure 1 * | 1-15 | |
| X | SÖDERSTRÖM S ET AL: "Recombinant human beta-nerve growth factor (NGF): biological activity and properties in an enzyme immunoassay." JOURNAL OF NEUROSCIENCE RESEARCH DEC 1990, vol. 27, no. 4, December 1990 (1990-12), pages 665-677, XP002520416 ISSN: 0360-4012 * abstract * * page 666, column 1, paragraph 3 - page 667, column 1, paragraph 2 * * figure 3 * | 14 | |
| X | US 2005/084883 A1 (MAITRA ANIRBAN [US] ET AL) 21 April 2005 (2005-04-21) * claims 28,29 * | 14 | |
| X | WO 2006/113880 A (UNIV COLUMBIA [US]; KIRMAN IRENA [US]; WHELAN RICHARD L [US]) 26 October 2006 (2006-10-26) * claims 14-16 * | 14 | |
| A | US 2007/092911 A1 (BUECHLER KENNETH F [US] ET AL) 26 April 2007 (2007-04-26) * claims 1-5,15,20 * | 1-15 | |

TECHNICAL FIELDS SEARCHED (IPC)

-/--

The present search report has been drawn up for all claims

| Place of search | Date of completion of the search | Examiner |
|---|---|---|
| The Hague | 27 March 2009 | van der Kooij, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 08 16 4495

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EMSLEY HEDLEY C A ET AL: "Clinical outcome following acute ischaemic stroke relates to both activation and autoregulatory inhibition of cytokine production." BMC NEUROLOGY 2007, vol. 7, 2007, page 5, XP002520417 ISSN: 1471-2377 * abstract * | 1-13,15 | |
| X | ANONYMOUS: "Quantikine- human sTNF RI/TNFRSF1A Immunoassay" INTERNET ARTICLE, [Online] 21 December 2007 (2007-12-21), XP002520418 Retrieved from the Internet: URL:http://www.rndsystems.com/pdf/drt100.pdf> [retrieved on 2008-03-20] * the whole document * | 14 | |
| X | EP 1 930 445 A (TORAY INDUSTRIES [JP]; UNIV KYOTO [JP]) 11 June 2008 (2008-06-11) * claims 20-31 * | 14 | TECHNICAL FIELDS SEARCHED (IPC) |
| A | LOSY JACEK ET AL: "CXCL1 (GRO-alpha) chemokine level is increased in CSF of acute ischaemic stroke patients and has a predictive value for short term stroke outcome" NEUROLOGY, LIPPINCOTT WILLIAMS & WILKINS, PHILADELPHIA, US, vol. 64, no. 6, Suppl. 1, 1 March 2005 (2005-03-01), page A399, XP008098554 ISSN: 0028-3878 * abstract * | 1-15 | |

The present search report has been drawn up for all claims

| Place of search | Date of completion of the search | Examiner |
|---|---|---|
| The Hague | 27 March 2009 | van der Kooij, M |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

**Application Number**

EP 08 16 4495

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

1. claims: 1-15 (all partially)

    A method of diagnosing a stroke mimicking condition or for the differential diagnosis of stroke or stroke mimicking conditions using IL-17 as diagnostic target. A method for determining the efficacy of a stroke therapy or stroke therapy comprising determining the level of IL-17 in a sample of the subject. Methods of screening for compounds suitable for treating stroke or stroke mimicking condition by monitoring IL-17 levels after administration of said compounds. Kit comprising a reagent for the analysis of IL-17. Use of kit comprising regent for analysis of IL-17 for diagnosing/prognosing of stroke or stroke mimicking condition.
    ---

2. claims: 1-15 (all partially)

    The method of diagnosing a stroke mimicking condition or for the differential diagnosis of stroke or stroke mimicking conditions using FasL as diagnostic target. A method for determining the efficacy of a stroke therapy or stroke therapy comprising determining the level of FasL in a sample of the subject. Methods of screening for compounds suitable for treating stroke or stroke mimicking condition by monitoring FasL levels after administration of said compounds. Kit comprising a reagent for the analysis of FasL. Use of kit comprising regent for analysis of FasL for diagnosing/prognosing of stroke or stroke mimicking condition.
    ---

3. claims: 1-15 (all partially)

    The method of diagnosing a stroke mimicking condition or for the differential diagnosis of stroke or stroke mimicking conditions using bNGF as diagnostic target. A method for determining the efficacy of a stroke therapy or stroke therapy comprising determining the level of bNGF in a sample of the subject. Methods of screening for compounds suitable for treating stroke or stroke mimicking condition by monitoring bNGF levels after administration of said compounds. Kit comprising a reagent for the analysis of bNGF. Use of kit comprising regent for analysis of bNGF for diagnosing/prognosing of stroke or stroke mimicking condition.
    ---

4. claims: 1-15 (all partially)

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

The method of diagnosing a stroke mimicking condition or for the differential diagnosis of stroke or stroke mimicking conditions using IGFBP3 as diagnostic target. A method for determining the efficacy of a stroke therapy or stroke therapy comprising determining the level of IGFBP3 in a sample of the subject. Methods of screening  for compounds suitable for treating stroke or stroke mimicking condition by monitoring IGFBP3 levels after administration of said compounds. Kit comprising a reagent for the analysis of IGFBP3. Use of kit comprising regent for analysis of IGFBP3 for diagnosing/prognosing of stroke or stroke mimicking condition.

---

5. claims: 1-15 (all partially)

The method of diagnosing a stroke mimicking condition or for the differential diagnosis of stroke or stroke mimicking conditions using TNFR-1 as diagnostic target. A method for determining the efficacy of a stroke therapy or stroke therapy comprising determining the level of TNFR-1 in a sample of the subject. Methods of screening  for compounds suitable for treating stroke or stroke mimicking condition by monitoring TNFR-1 levels after administration of said compounds. Kit comprising a reagent for the analysis of TNFR-1. Use of kit comprising regent for analysis of TNFR-1 for diagnosing/prognosing of stroke or stroke mimicking condition.

---

6. claims: 1-15 (all partially)

The method of diagnosing a stroke mimicking condition or for the differential diagnosis of stroke or stroke mimicking conditions using GroA as diagnostic target. A method for determining the efficacy of a stroke therapy or stroke therapy comprising determining the level of GroA in a sample of the subject. Methods of screening  for compounds suitable for treating stroke or stroke mimicking condition by monitoring GroA levels after administration of said compounds. Kit comprising a reagent for the analysis of GroA. Use of kit comprising regent for analysis of GroA for diagnosing/prognosing of stroke or stroke mimicking condition.

---

## ANNEX TO THE EUROPEAN SEARCH REPORT
## ON EUROPEAN PATENT APPLICATION NO.

EP 08 16 4495

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2009

| Patent document cited in search report | | Publication date | Patent family member(s) | | Publication date |
|---|---|---|---|---|---|
| US 2003199000 | A1 | 23-10-2003 | US | 2003119064 A1 | 26-06-2003 |
| WO 2004059293 | A | 15-07-2004 | AU | 2003302340 A1 | 22-07-2004 |
| | | | CA | 2511501 A1 | 15-07-2004 |
| | | | EP | 1587955 A2 | 26-10-2005 |
| | | | JP | 2006526140 T | 16-11-2006 |
| US 6197525 | B1 | 06-03-2001 | US | 6096305 A | 01-08-2000 |
| | | | US | 6072033 A | 06-06-2000 |
| | | | US | 6191104 B1 | 20-02-2001 |
| | | | US | 6100235 A | 08-08-2000 |
| | | | US | 6072037 A | 06-06-2000 |
| US 6946255 | B1 | 20-09-2005 | AT | 327258 T | 15-06-2006 |
| | | | AU | 716731 B2 | 02-03-2000 |
| | | | AU | 5014196 A | 08-10-1996 |
| | | | CA | 2215905 A1 | 26-09-1996 |
| | | | DE | 69636170 T2 | 02-11-2006 |
| | | | EP | 0872488 A1 | 21-10-1998 |
| | | | WO | 9629350 A1 | 26-09-1996 |
| | | | JP | 3451092 B2 | 29-09-2003 |
| US 2004219509 | A1 | 04-11-2004 | NONE | | |
| US 2004237124 | A1 | 25-11-2004 | US | 2007212357 A1 | 13-09-2007 |
| US 2005084883 | A1 | 21-04-2005 | NONE | | |
| WO 2006113880 | A | 26-10-2006 | NONE | | |
| US 2007092911 | A1 | 26-04-2007 | NONE | | |
| EP 1930445 | A | 11-06-2008 | CA | 2621060 A1 | 08-03-2007 |
| | | | CN | 101297046 A | 29-10-2008 |
| | | | WO | 2007026895 A1 | 08-03-2007 |
| | | | KR | 20080046693 A | 27-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040219509 A
- US 6143576 A
- US 6113855 A
- US 6019944 A
- US 5985579 A
- US 5947124 A
- US 5939272 A
- US 5922615 A
- US 5885527 A
- US 5851776 A

- US 5824799 A
- US 5679526 A
- US 5525524 A
- US 5480792 A
- US 5631171 A
- US 5955377 A
- US 6432662 B
- US 5571698 A, Ladner
- US 6057098 A

**Non-patent literature cited in the description**

- **Nor AM et al.** The Recognition of Stroke in the Emergency Room (ROSIER) scale: development and validation of a stroke recognition instrument. *Lancet Neurol.,* November 2005, vol. 4 (11), 727-34
- **Hand PJ et al.** Distinguishing between stroke and mimic at the bedside: the brain attack study. *Stroke,* March 2006, vol. 37 (3), 769-75
- **Dowdy ; Wearden.** Statistics for Research. John Wiley & Sons, 1983
- **Li HL et al.** IL-17 and IFN-gamma mRNA expression is increased in the brain and systemically after permanent middle cerebral artery occlusion in the rat. *J Neuroimmunol.,* 2001, vol. 116 (1), 5-14
- **Li GZ et al.** Expression of interleukin-17 in ischemic brain tissue. *Scand J Immunol.,* November 2005, vol. 62 (5), 481-6
- **Kostulas N et al.** Increased IL-1beta, IL-8, and IL-17 mRNA expression in blood mononuclear cells observed in a prospective ischemic stroke study. *Stroke,* October 1999, vol. 30 (10), 2174-9
- **Tarkowski E et al.** Intrathecal expression of proteins regulating apoptosis in acute stroke. *Stroke,* February 1999, vol. 30 (2), 321-7
- **Liu L et al.** FasL shedding is reduced by hypothermia in experimental stroke. *J Neurochem,* 13 May 2008
- **Gratas C et al.** Fas ligand expression in glioblastoma cell lines and primary astrocytic brain tumors. *Brain Pathol.,* July 1997, vol. 7 (3), 863-9
- **Lorez et al.** Nerve growth factor increases in adult rat brain after hypoxic injury. *Neurosci. Lett.,* vol. 98, 339-344

- **Stanzani et al.** Nerve growth factor and transforming growth factor-beta serum levels in acute stroke patients. Possible involvement of neurotrophins in cerebrovascular disease. *Cerebrovasc Dis.,* 2001, vol. 12 (3), 240-4
- **Reynolds MA et al.** Early biomarkers of stroke. *Clin Chem.,* October 2003, vol. 49 (10), 1733-9
- **Schwab S et al.** Plasma insulin-like growth factor I and IGF binding protein 3 levels in patients with acute cerebral ischemic injury. *Stroke,* September 1997, vol. 28 (9), 1744-8
- **Wilczak N et al.** Intravenous tissue plasminogen activator in patients with stroke increases the bioavailability of insulin-like growth factor-1. *Stroke,* September 2006, vol. 37 (9), 2368-71
- **Lönn S et al.** Glioma risk in relation to serum levels of insulin-like growth factors. *Cancer Epidemiol Biomarkers Prev.,* April 2007, vol. 16 (4), 844-6
- **Unterman TG et al.** Production of insulin-like growth factor-binding proteins by human central nervous system tumors. *Cancer Res.,* 01 June 1991, vol. 51 (11), 3030-6
- **Dziewulska D ; Mossakowski MJ.** Cellular expression of tumor necrosis factor a and its receptors in human ischemic stroke. *Clin Neuropathol.,* January 2003, vol. 22 (1), 35-40
- **Elneihoum AM et al.** Leukocyte activation detected by increased plasma levels of inflammatory mediators in patients with ischemic cerebrovascular diseases. *Stroke,* October 1996, vol. 27 (10), 1734-8
- **Losy J et al.** CXCL1 (GRO-alpha) chemokine in acute ischaemic stroke patients. *Folia Neuropathol.,* 2005, vol. 43 (2), 97-102

- **Zhou Y et al.** The chemokine GRO-alpha (CXCL1) confers increased tumorigenicity to glioma cells. *Carcinogenesis,* 20 July 2005, vol. 26 (12), 2058-68
- **Ng ; Ilag.** *J. Cell Mol. Med.,* 2002, vol. 6, 329-340
- **Lash GE et al.** Comparison of three multiplex cytokine analysis systems: Luminex, SearchLight and FAST Quant. *J Immunol Methods,* 20 February 2006, vol. 309 (1-2), 205-8
- **Tietz.** Textbook of Clinical Chemistry. W. B. Saunders and Company, 496
- Meth. Enzymol. Guide to Protein Purification. 1990, vol. 182
- Meth. Enzymol. Solid Phase Peptide Synthesis. 1997, vol. 289
- **Kiso et al.** *Chem. Pharm. Bull. (Tokyo),* 1990, vol. 38, 1192-99
- **Mostafavi et al.** *Biomed. Pept. Proteins Nucleic Acids,* 1995, vol. 1, 255-60
- **Fujiwara et al.** *Chem. Pharm. Bull. (Tokyo),* 1996, vol. 44, 1326-31
- Antibodies, A Laboratory Manual. Cold Spring Harbor Laboratory, 1988
- Antibody Engineering: A Practical Approach. Oxford University Press, 1995
- *J. Immunol.,* 1992, vol. 149, 3914-3920
- **Cwirla et al.** *Proc. Natl. Acad. Sci. USA,* 1990, vol. 87, 6378-82
- **Devlin et al.** *Science,* 1990, vol. 249, 404-6
- **Scott ; Smith.** *Science,* 1990, vol. 249, 386-88
- **Chomczynski et al.** *Anal. Biochem.,* 1987, vol. 162, 156
- **Chomczynski P.** *Biotechniques,* 1993, vol. 15, 532
- **Rosell A et al.** A matrix metalloproteinase protein array reveals a strong relation between MMP-9 and MMP-13 with diffusion-weighted image lesion increase in human stroke. *Stroke,* July 2005, vol. 36 (7), 1415-20